# EUROPEAN PATENT SPECIFICATION

(11) **EP 3 964 217 B1**
(45) Date of publication and mention of the grant of the patent: **07.10.2026**
(21) Application number: 20886184.9
(22) Date of filing: 19.08.2020
(51) Int. Cl.: A61K 31/7034, A61P 35/00, A61K 31/352, A61K 31/404, A61K 31/44, A61K 31/4439, A61K 31/47, A61K 31/4709, A61K 31/496, A61K 31/5025, A61K 31/506, A61K 31/517, A61K 31/519, A61K 31/53, A61K 31/5377, A61K 31/55

(54) **SOTAGLIFLOZIN FOR USE IN TREATING LUNG CANCER RESISTANT TO GEFITINIB**
SOTAGLIFLOZIN ZUR VERWENDUNG IN DER BEHANDLUNG VON GEFITINIB-RESISTENTEM LUNGENKREBS
SOTAGLIFLOZINE DESTINÉE À ÊTRE UTILISÉE DANS LE TRAITEMENT DU CANCER DU POUMON RÉSISTANT AU GÉFITINIB

(30) Priority: 06.07.2020 CN 202010643578
(43) Date of publication of application: 09.03.2022
(73) Proprietor: Newish Technology (Beijing) Co., Ltd., Economic & Technical Development Zone Beijing 100176 (CN)
(72) Inventor: SUN, Zhongjie, Beijing 100176 (CN); WANG, Xiaofang, Beijing 100176 (CN); QI, Hailong, Beijing 100176 (CN)
(74) Representative: Studio Torta S.p.A.
(86) International application number: PCT/CN2020/109966
(87) International publication number: WO 2022/007134

(56) References cited:
- WO-A1-2018/026673
- WO-A1-2018/073154
- WO-A1-2019/201752
- WO-A1-2019/201752
- WO-A1-2019/232403
- CN-A- 109 843 279
- US-A1- 2014 336 129
- US-A1- 2014 371 254
- US-B2- 9 597 299
- CHEN CHIA-HUNG ET AL: "PKC[delta]-mediated SGLT1 upregulation confers the acquired resistance of NSCLC to EGFR TKIs", ONCOGENE, NATURE PUBLISHING GROUP UK, LONDON, vol. 40, no. 29, 21 June 2021 (2021-06-21), pages 4796 - 4808, XP037517462, ISSN: 0950-9232, [retrieved on 20210621], DOI: 10.1038/S41388-021-01889-0
- CLAUDIO SCAFOGLIO, BRUCE A. HIRAYAMA, VLADIMIR KEPE, JIE LIU, CHIARA GHEZZI, NAGICHETTIAR SATYAMURTHY, NEDA A. MOATAMED, JIAOTI HU: "Functional expression of sodium-glucose transporters in cancer", PROCEEDINGS OF THE NATIONAL ACADEMY OF SCIENCES, NATIONAL ACADEMY OF SCIENCES, vol. 112, no. 30, 28 July 2015 (2015-07-28), pages E4111 - E4119, XP055461914, ISSN: 0027-8424, DOI: 10.1073/pnas.1511698112
- JIANGONG REN, LAKSHMI R. BOLLU, FEI SU, GUANG GAO, LEI XU, WEI-CHIEN HUANG, MIEN-CHIE HUNG, ZHANG WEIHUA: "EGFR-SGLT1 interaction does not respond to EGFR modulators, but inhibition of SGLT1 sensitizes prostate cancer cells to EGFR tyrosine kinase inhibitors", THE PROSTATE, WILEY-LISS, NEW YORK, NY., US, vol. 73, no. 13, 1 September 2013 (2013-09-01), US , pages 1453 - 1461, XP055306982, ISSN: 0270-4137, DOI: 10.1002/pros.22692

## Description

### CROSS REFERENCE TO RELATED APPLICATIONS

This application claims the priority of Chinese Patent Application No. 202010643578.4 filed at the Chinese Patent Office on July 6, 2020, titled with "USE OF COMPOUND OR PHARMACEUTICALLY ACCEPTABLE SALT, DIMER OR TRIMER THEREOF IN MANUFACTURE OF MEDICAMENT FOR TREATING CANCER.

### FIELD

The present disclosure relates to the field of medicine technology, and in particular to a compound or a pharmaceutically acceptable salt thereof for use in the treatment of lung cancer.

### BACKGROUND

### Cancer Epidemiology

Non-communicable diseases are the main cause of death in the world, and cancer is the disease with the highest fatality rate among non-communicable diseases, bringing a heavy burden to the social health and medical system. Traditional cancer treatment is mainly surgery, radiotherapy and chemotherapy, and chemotherapy is the main treatment for advanced cancer. Classical chemotherapy has serious side effects due to poor targeting. The emergence of targeted chemotherapy drug such as Gleevec has greatly reduced the pain caused by chemotherapy to patients. Targeted drugs are designed based on the different growth characteristics and expression of cancer cells compared with normal cells. For example, Gleevec specifically targets the constitutively activated tyrosine kinases in chronic myelogenous leukemia to achieve a good therapeutic effect (Flynn and Gerriets, 2020). Another distinguishing feature of cancer cells compared with normal cells is the change in metabolism. In order to meet the needs of cell components for rapid proliferation and maintain the energy supply needed for survival, cancer cells prefer to use glucose by aerobic glycolysis, which is called the Warburg effect (Warburg, 1956).

Aerobic glycolysis cannot fully oxidize glucose to produce ATP, but can produce a large amount of intermediate metabolites for DNA and protein synthesis to promote cancer cell proliferation. Therefore, it is feasible to target the sugar metabolism of tumor cells to inhibit the proliferation of cancer cells (Kroemer and Pouyssegur, 2008).

### Sotagliflozin and cancer treatment

Cancer cells absorb glucose from the external environment mainly through glucose transporters. Glucose transporters are divided into two major families, one of which is the GLUT family that transports glucose along the glucose concentration gradient by assisting diffusion, and the other is the sodium-glucose co-transporter SGLT family that co-transports sodium ions for glucose absorption and actively transports glucose from the external for cell consumption by consuming ATP (Navale and Paranjape, 2016). The two main members of the SGLT family are SGLT1 and SGLT2. SGLT2 is mainly distributed at the front end of the proximal convoluted tubule of the kidney, and reabsorbs more than 97% of the glucose from the original urine into the blood through active transport, while SGLT1 is mainly distributed in the epithelial cells of the small intestine chorion and the distal end of the proximal convoluted tubules of the kidney, and through active transport absorbs glucose from food in the intestine and the remaining 3% glucose in the original urine after the absorption by SGLT2 (Dominguez Rieg and Rieg, 2019). Due to the important role of SGLT1 and SGLT2 in sugar absorption and reabsorption, they have become ideal targets for diabetes treatment. Currently Empagliflozin, Canagliflozin and Dapagliflozin targeting SGLT2 have shown good therapeutic effects in the treatment of type 2 diabetes, and the effect of reducing cardiovascular disease. Migliflozin, which targets SGLT1 alone, has entered the clinical research phase. Sotagliflozin, which targets both SGLT1 and SGLT2, has been approved for marketing in the EU.

WO2019/201752 A1 discloses a pharmaceutical combination comprising an AOC3 inhibitor and an SGLT2 inhibitor.

US9597299 B2 discloses a method for treating gefitinib-resistant non-small-cell lung cancer (NSCLC) comprising administering an effective amount of a resveratrol analog, (Z)3,4,5,4'-tetramethoxystilbene (TMS), to a subject in need thereof. This document also relates to a method for inducing apoptosis in gefitinib-resistant NSCLC cells comprising contacting the resveratrol analogue to the cells at an effective amount.

US2014/371254 A1 discloses a method of preventing and treating Gefitinib-resistant non-small-cell lung cancer, comprising administering an effective amount of an alkaloid and a pharmaceutical composition comprising an alkaloid admixed with a pharmaceutical carrier for treating Gefitinib-resistant non-small-cell lung cancer.

There is now no report on the use of Sotagliflozin in the treatment of cancer.

### SUMMARY

In view of this, the technical problem to be solved by the present disclosure is to provide the use of Sotagliflozin in the manufacture of a medicament for treating cancer.

The present disclosure provides use of a compound of formula I 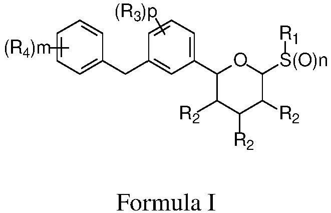 wherein:
R₁ is hydrogen, or C₁₋₁₀-alkyl, C₁₋₅-cycloalkyl or 5-membered heterocyclic ring optionally substituted with one or more R_{1A};
each R_{1A} is independently amino, ester, amide, thiol, carboxylic acid, cyano, halogen, hydroxyl, or C₁₋₄-alkoxy, C₁₋₅-cycloalkyl or 5-membered heterocyclic ring optionally substituted with one or more R_{1B}; each R_{1B} is independently C₁₋₄-alkyl, halogen or hydroxy; n is 0, 1 or 2;
each R₂ is independently F or OR_{2A}, wherein each R_{2A} is independently hydrogen, C₁₋₄-alkyl or acyl;
each R₃ is independently halogen, hydroxy, or C₁₋₁₀-alkyl or C₁₋₁₀-alkoxy optionally substituted with one or more R_{3A};
each R_{3A} is independently amino, ester, amide, thiol, carboxylic acid, cyano, halogen, hydroxyl, or C₁₋₄-alkoxy, C₁₋₅-cycloalkyl or 5-membered heterocyclic ring optionally substituted with one or more R_{3B}; each R_{3B} is independently C₁₋₄-alkyl, amino, cyano, halogen or hydroxyl; p is 0, 1 or 2;
each R₄ is independently R_{4A}, -N(R_{4A})(R_{4B}), -OR_{4A}, -SR_{4A}, -S(O)R_{4A} or-S(O)₂R_{4A};
R_{4A} is C₄₋₂₀-alkyl or 4-20 membered heteroalkyl optionally substituted with one or more R_{4C} and optionally attached to another R_{4A} to provide a dimer or trimer; R_{4B} is hydrogen or R_{4A}; each R_{4C} is independently amino, aminoacyl, azo, carbonyl, carboxyl, cyano, formyl, guanidino, halogen, hydroxyl, iminoacyl, imino, isothiocyanate, nitrile, nitro, nitroso, nitroxyl, oxy, alkylthio, sulfinyl, sulfonyl, thioaldehyde, thiocyanate, thioketone, thiourea, urea or X₁, X₁-L₁-X₂ or X₁-L₁-X₂-L₂-X₃, wherein each of X₁, X₂ and X₃ is independently C₁₋₄-alkyl, C₁₋₆-cycloalkyl, 5- or 6-membered heterocyclic or aryl optionally substituted with one or more R_{4D}, and each of L₁ and L₂ is independently C₁₋₆-alkyl or 1-10 membered heteroalkyl optionally substituted with one or more R_{4E}; each R_{4D} is independently R_{4E}, or C₁₋₆-alkyl optionally substituted with one or more R_{4E}; each R_{4E} is independently amino, aminoacyl, azo, carbonyl, carboxyl, cyano, formyl, guanidino,
halogen, hydroxyl, iminoacyl, imino, isothiocyanate, nitrile, nitro, nitroso, nitroxyl, oxo, alkylthio, sulfinyl, sulfonyl, thioaldehyde, thiocyanate, thioketone or urea; and m is 1, 2 or 3;
or a pharmaceutically acceptable salt, dimer or trimer thereof in the manufacture of a medicament for treating cancer.

In the present disclosure, the cancer treated by the compound of formula I or a pharmaceutically acceptable salt, dimer or trimer thereof includes bladder cancer, blood cancer, bone cancer, brain cancer, breast cancer, central nervous system cancer, cervical cancer, colon cancer, endometrial cancer, esophageal cancer, gallbladder cancer, gastrointestinal cancer, external genital cancer, urogenital cancer, head cancer, kidney cancer, laryngeal cancer, liver cancer, muscle tissue cancer, neck cancer, oral or nasal mucosal cancer, ovarian cancer, prostate cancer, skin cancer, spleen cancer, small bowel cancer, large bowel cancer, stomach cancer, testicular cancer and/or thyroid cancer.

In an embodiment, R₁ is methyl, n=0; each R₂ is -OH; p=1, R₃ is Cl; m=1, and R4 is ethoxy.

The compound of Formula I is Sotagliflozin with the structure as shown in Formula II.

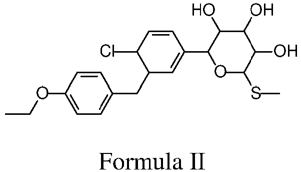

In the present disclosure, the treatment includes inhibiting tumor cell proliferation and/or inhibiting tumor volume. In some examples, lung cancer A549 cells, liver cancer HepG2 cells, prostate cancer DU145 cells, breast cancer MCF-7 cells, esophageal cancer KYSE30 cells, gastric cancer HGC-27 cells, cholangiocarcinoma RBE cells, ovarian cancer SKOV3 cells, and cervical cancer HeLa cells were used to verify the inhibitory effect of Sotagliflozin on tumor cells, and the cell experiments showed that Sotagliflozin had a certain inhibitory effect on tumor cells, with an IC₅₀ of 71.57 µM, 115.7 µM, 40.77 µM, 64.84 µM, 82.27 µM, 48.54 µM, 182.5 µM, 84.49 µM and 82.11 µM, respectively. In animal experiments, Sotagliflozin showed a good inhibitory effect on tumor volume, which was very significantly different from that of the solvent control group, p<0.05.

The present disclosure also provides use of a compound of formula I or a pharmaceutically acceptable salt, dimer or trimer thereof in the manufacture of a preparation for reversing the resistance to an anti-tumor drug. In some specific embodiments, the present disclosure provides use of Sotagliflozin in the manufacture of a preparation for reversing the resistance to an anti-tumor drug.

The present disclosure also provides a method for reversing the resistance to an anti-tumor drug, comprising administering a compound of formula I or a pharmaceutically acceptable salt, dimer or trimer thereof. In some specific embodiments, the present disclosure also provides a method for reversing the resistance to an anti-tumor drug, comprising administering Sotagliflozin.

In the present disclosure, the compound of formula I or a pharmaceutically acceptable salt, dimer or trimer thereof is capable of reversing the resistance to an anti-tumor drug, and the anti-tumor drug is a tyrosine kinase activity inhibitor.

In the present disclosure, the tyrosine kinase activity inhibitor includes: EGFR inhibitor, c-Kit, c-Met, c-Ret, Raf, PDGFR, BTK, PKA/C, FGFR inhibitor or VEGFR inhibitor.

In some embodiments, the EGFR inhibitor includes: Gefitinib, Erlotinib, Afatinib, Lapatinib ditosylate, Genistein, Lapatinib, Sapitinib, Daphnetin, Dacomitinib, Varlitinib, Icotinib, Lidocaine hydrochloride, Osimertinib mesylate, Osimertinib, Poziotinib, Nazartinib, AZD3759, Olmutinib, Avitinib, Neratinib, Lazertinib;
The c-Met inhibitor includes: Cabozantinib;
The PKA/C inhibitor includes: Daphnetin;
The BTK inhibitor includes: Olmutinib;
The c-Ret inhibitor includes: Regorafenib monohydrate and Regorafenib;
The Raf inhibitor includes: Regorafenib monohydrate;
The FGFR inhibitor includes: 4-[(1E)-2-[5-[(1R)-1-(3,5-dichloro-4-pyridyl)ethoxy]-1H - indazol-3-yl]vinyl]-1H-pyrazole-1-ethanol(LY2874455); Nintedanib, Nintedanib Ethanesulfonate, Ponatinib, Brivanib, Brivanib Alaninate;
The c-Kit inhibitor includes: Axitinib, Pazopanib, Pazopanib HCl, Regorafenib Monohydrate, Sunitinib Malate, Sunitinib, Sitravatinib, Telatinib;
The PDGFR inhibitor includes: Axitinib, Tivozanib, Telatinib, Nintedanib, Nintedanib Ethanesulfonate Salt, Pazopanib, Pazopanib HCl, Ponatinib;
The VEGFR inhibitors include: Apatinib, Axitinib, Nintedanib, Cediranib, Pazopanib HCl, Sunitinib Malate, Brivanib, Cabozantinib, Brivanib Alaninate, Lenvatinib, Regorafenib, ENMD-2076, ENMD-2076 L-(+)-Tartaric acid, Tivozanib, Ponatinib, Fruquintinib, Telatinib, Taxifolin, Pazopanib, Cabozantinib malate, Vitamin E, Regorafenib Monohydrate, Nintedanib Ethanesulfonate Salt, Lenvatinib Mesylate, Cediranib Maleate, LY2874455, Sunitinib, Sitravatinib, Anlotinib, Sorafenib, Vandetanib and Bevacizumab and other monoclonal antibodies targeting VEGFR.

In the present disclosure, the anti-tumor drugs used to verify the efficacy of Sotagliflozin for reversing the resistance to an anti-tumor drug include at least one of ENMD-2076, Tivozanib, Genistein, Ponatinib, Daphnetin, Dacomitinib, Varlitinib, Icotinib, Osimertinib mesylate, Osimertinib, Nazartinib, AZD3759, Anlotinib, Avitinib, or Lazertinib , Lidocaine hydrochloride, Y2874455, Axitinib, Nintedanib, Cediranib, Pazopanib HCl, Sunitinib Malate, Brivanib, Cabozantinib, Brivanib Alaninate, Lenvatinib, Regorafenib, ENMD-2076 L-(+)-Tartaric acid, Telatinib, Pazopanib, Cabozantinib malate, Regorafenib Monohydrate, Nintedanib Ethanesulfonate Salt, Lenvatinib Mesylate, Cediranib Maleate, Fruquintinib, Sunitinib, Olmutinib, Sitravatinib, Vandetanib, Gefitinib, Afatinib, Apatinib, Erlotinib, or Sorafenib, Taxifolin or Vitamin E.

In an example of the present disclosure, a cell line resistant to 60 µM Gefitinib was first constructed, and then a combination of 30 µM Gefitinib and Sotagliflozin was added to the Gefitinib resistant cell line obtained in the present disclosure. The cell line was effectively killed, it means that the combination of Sotagliflozin and Gefitinib reversed the resistance of tumor cells to Gefitinib.

The present disclosure also provides a medicament for treating cancer, comprising a compound of formula I or a pharmaceutically acceptable salt, dimer or trimer thereof, as well as pharmaceutically acceptable excipients.

The medicament of the present disclosure also comprises other drugs with anti-tumor effects, for example, tyrosine kinase activity inhibitors.

The medicament is administered orally, and its dosage forms include granules, pills, powders, tablets, capsules, oral solutions or syrups.

In some embodiments of the present disclosure, the capsule is a hard capsule or a soft capsule.

In some embodiments of the present disclosure, the tablet is an oral tablet or a buccal tablet.

Tablets refer to tablets for oral administration, and the active ingredients in most of such tablets are absorbed through the gastrointestinal tract to exert their effects, and active ingredients in some tablets act locally in the gastrointestinal tract. In some embodiments of the present disclosure, the tablets are ordinary compressed tablets, dispersible tablets, effervescent tablets, chewable tablets, coated tablets or sustained and controlled release tablets.

The medicament also comprises pharmaceutically acceptable auxiliary materials, including one of fruit powder, edible essence, sweetener, sour agent, filler, lubricant, preservative, suspending agent, food coloring, diluent, emulsifier, disintegrant and plasticizer, or a mixture thereof.

The present disclosure also provides a method for treating cancer, comprising administering the medicament of the present disclosure.

The present disclosure provides use of a compound of formula I or a pharmaceutically acceptable salt, dimer or trimer thereof in the manufacture of a medicament for treating canner. By testing the inhibitory effect on a variety of tumor cells, the results show that the compound of formula I has a certain inhibitory effect on tumor cells, with an IC50 of 40.77 µM-182.5 µM. In animal experiments using tumor-bearing mouse models produced from liver cancer cells and lung cancer cells, the compound of formula I showed a good inhibitory effect of tumor volume, which is very significantly different from that of the solvent control group, p<0.05.

The invention is defined in the claims. Any subject-matter which is not encompassed by the claims does not form part of the present invention and is provided for information, reference and/or comparison only.

Any reference to a method of treatment of the human or animal body by therapy using a certain compound or composition is to be understood as referring to said compound or composition for use in the treatment of said human or animal subject.

### BRIEF DESCRIPTION OF DRAWINGS

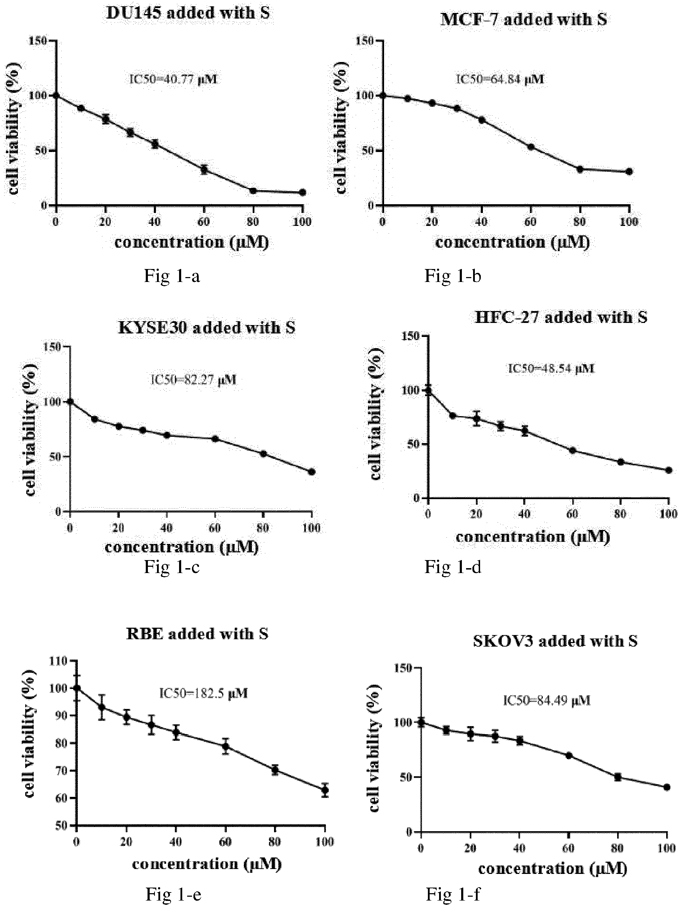

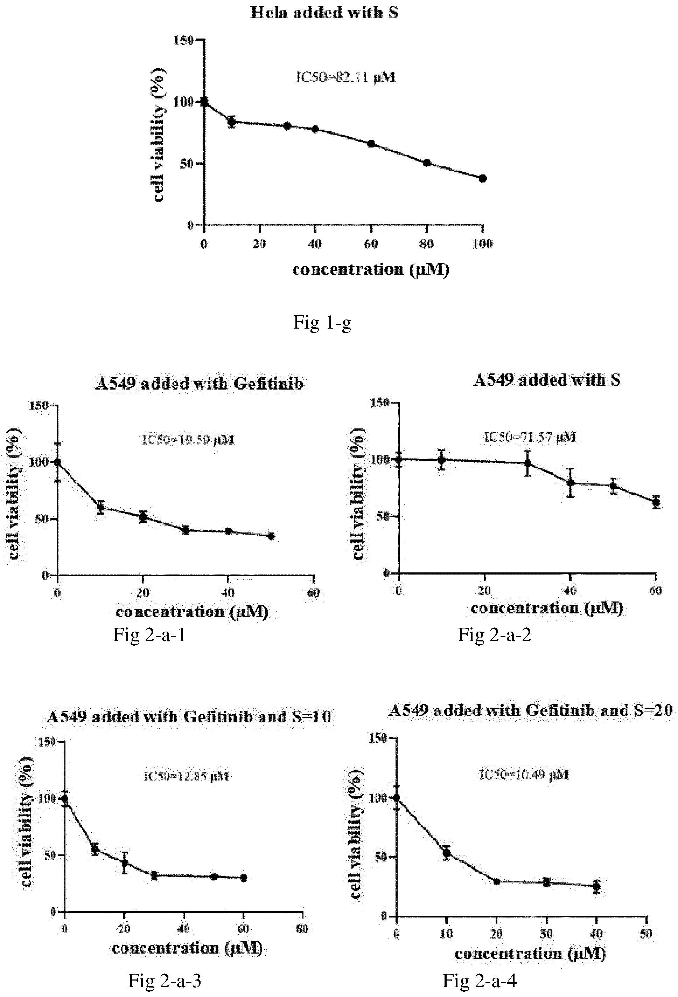

Figure 1 shows the killing effect of Sotagliflozin on prostate cancer DU145 cells; Figure 1-b shows the killing effect of Sotagliflozin on breast cancer MCF-7 cells; Figure 1-c shows the killing effect of Sotagliflozin on esophageal cancer KYSE30 cells; Figure 1-d shows the killing effect of Sotagliflozin on gastric cancer HGC-27 cells; Figure 1-e shows the killing effect of Sotagliflozin on cholangiocarcinoma RBE cells; Figure 1-f shows the killing effect of Sotagliflozin on ovarian cancer SKOV3 cells; and Figure 1-g shows the killing effect of Sotagliflozin on cervical cancer HeLa cells;

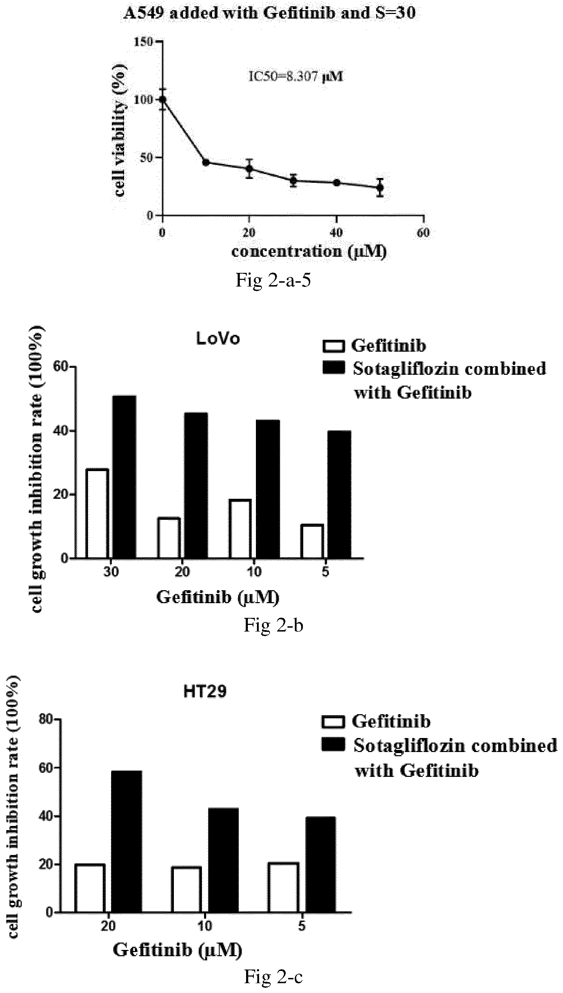

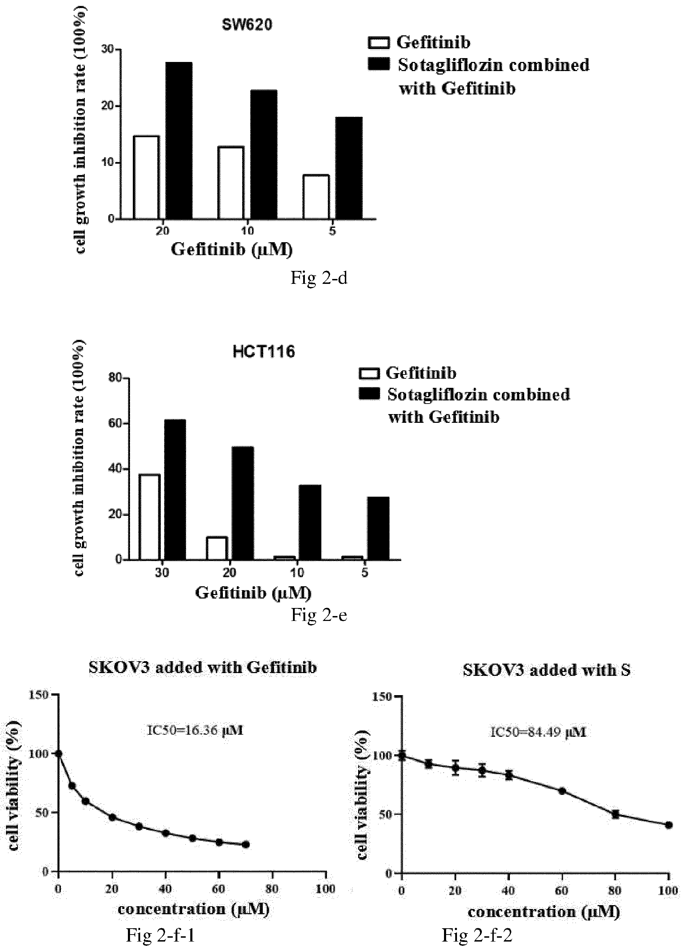

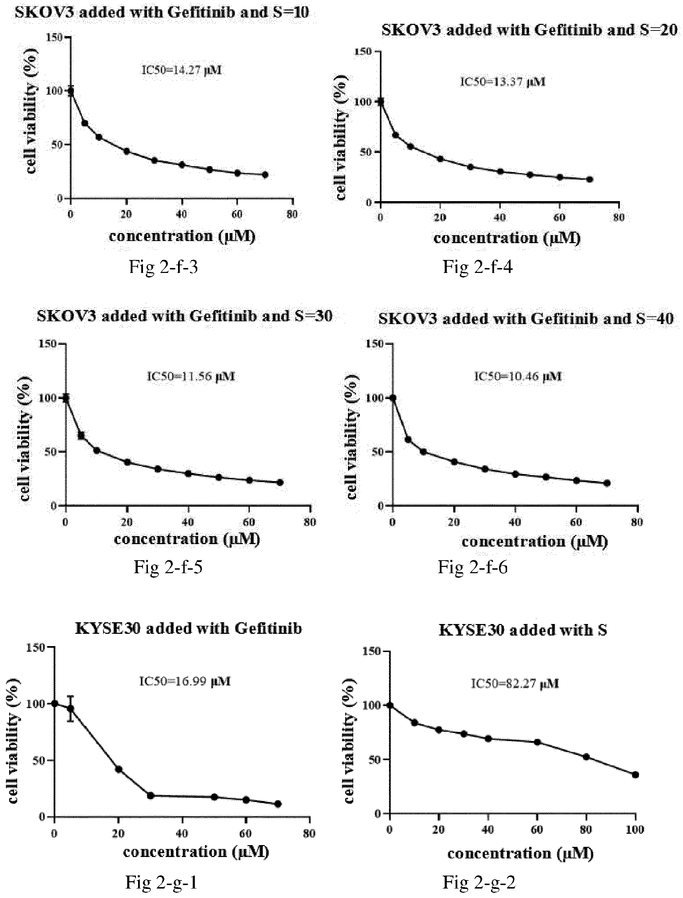

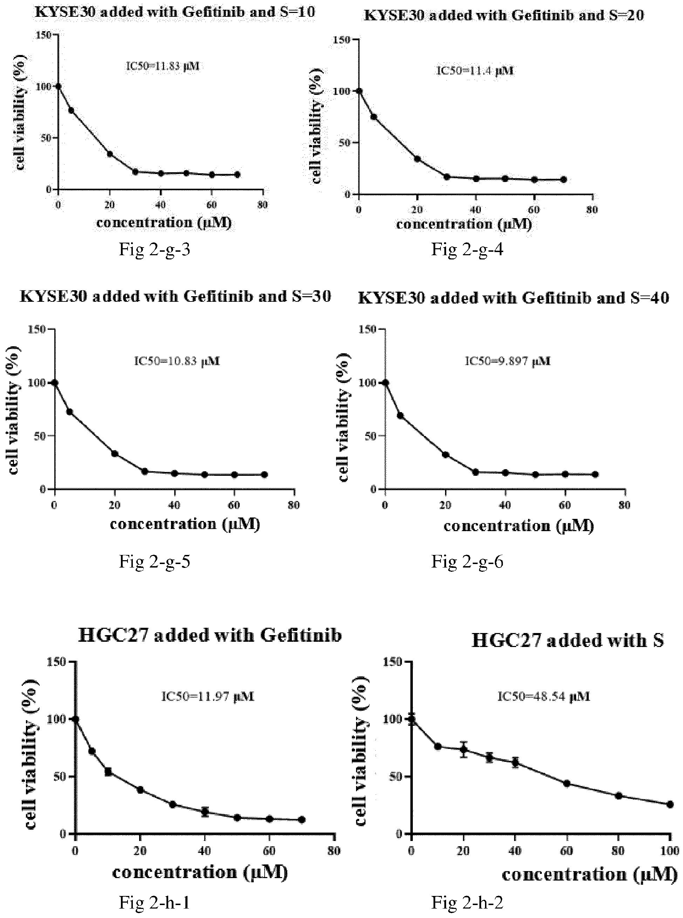

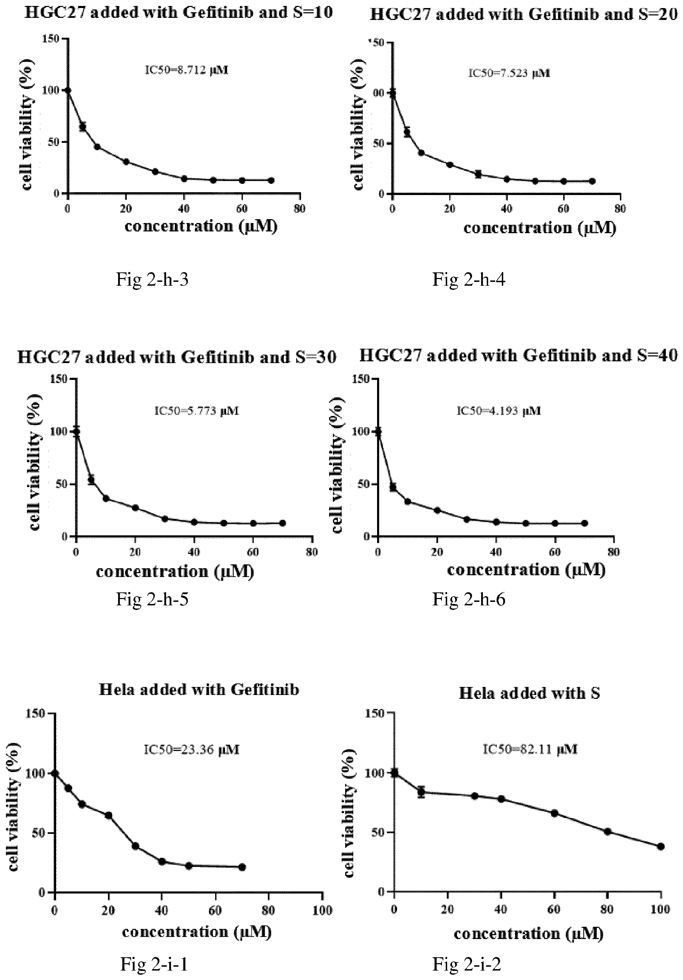

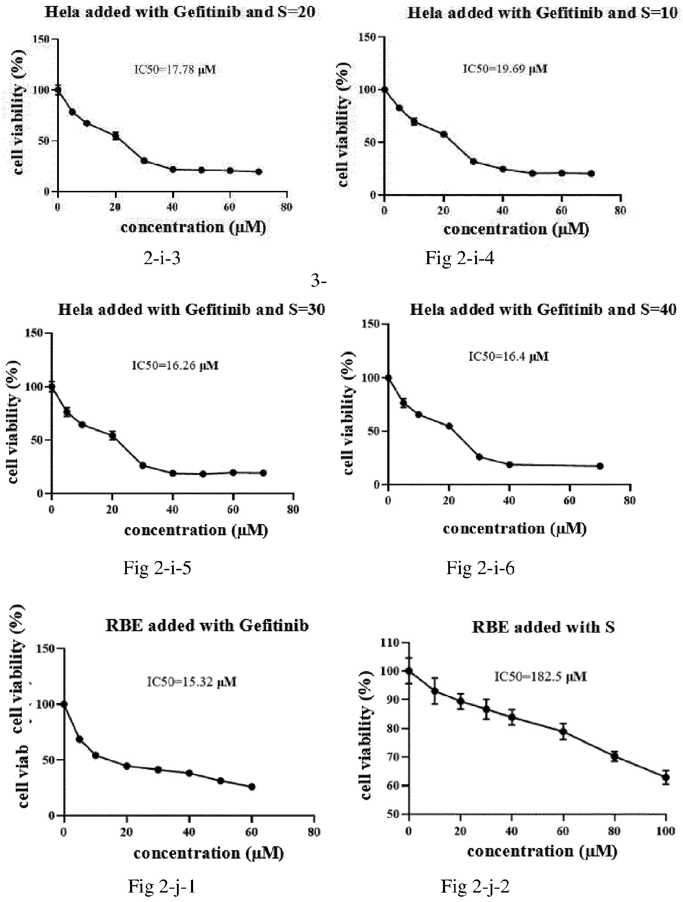

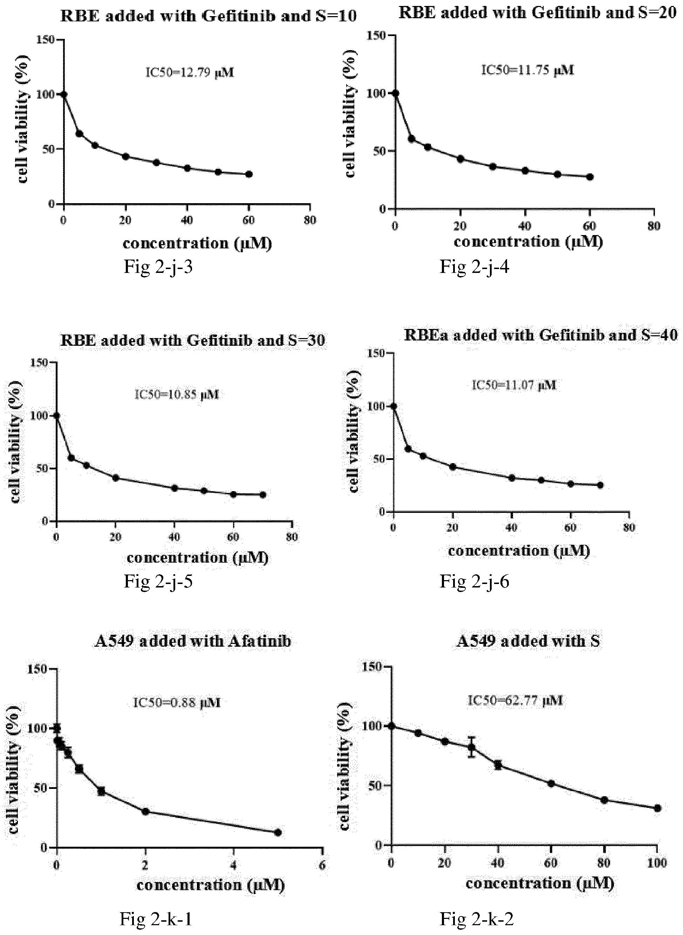

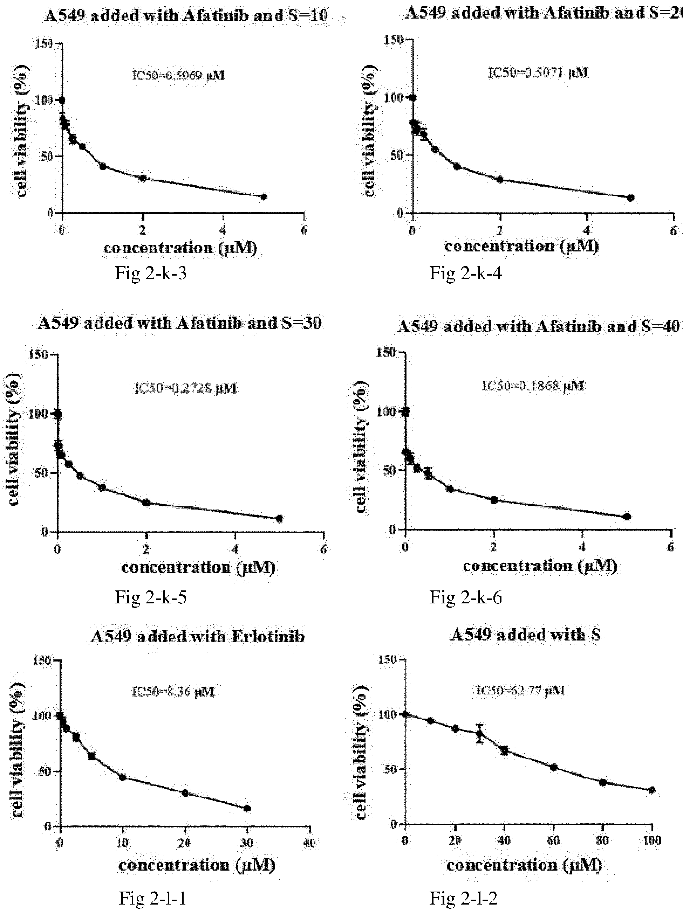

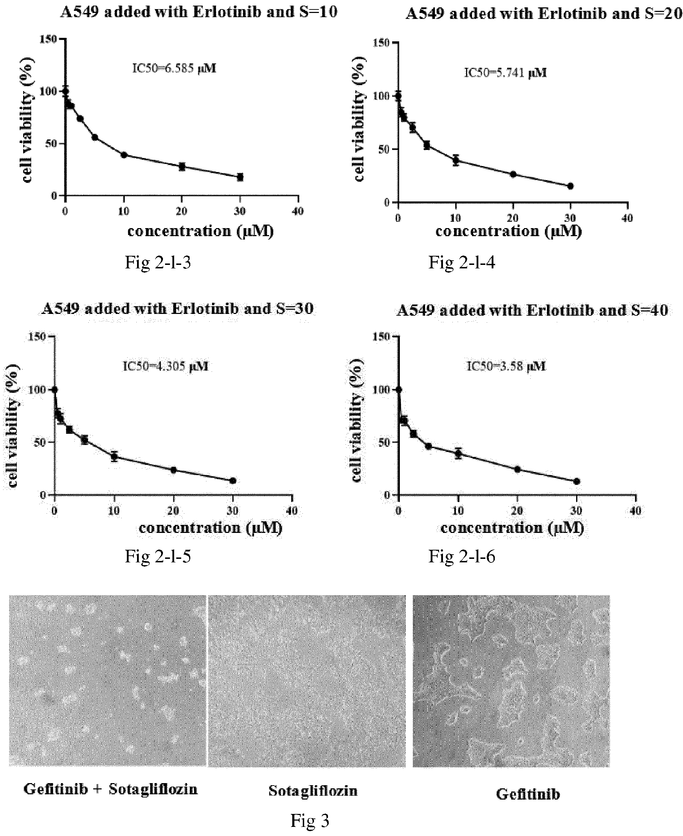

Figure 2-a-1 shows the inhibitory effect of different concentrations of Gefitinib on lung cancer cell line A549; Figure 2-a-2 shows the inhibitory effect of different concentrations of Sotagliflozin on lung cancer cell line A549; Figure 2-a-3 shows the inhibitory effect of different concentrations of Gefitinib+10 µM Sotagliflozin on lung cancer cell line A549 in test group 1; Figure 2-a-4 shows the effects of different concentrations of Gefitinib+20 µM Sotagliflozin on lung cancer cell line A549 in test group 2; Figure 2-a-5 shows the inhibitory effect of different concentrations of Gefitinib + 30µM Sotagliflozin on lung cancer cell line A549 in test group 3;
Figure 2-b shows the inhibition rate of Gefitinib alone and the combination of Sotagliflozin and Gefitinib on the growth of colorectal cancer cell line LoVo;
Figure 2-c shows the inhibition rate of Gefitinib alone and the combination of Sotagliflozin and Gefitinib on the growth of colorectal cancer cell line HT29;
Figure 2-d shows the inhibition rate of Gefitinib alone and the combination of Sotagliflozin and Gefitinib on the growth of colorectal cancer cell line SW620;
Figure 2-e shows the inhibition rate of Gefitinib alone and the combination of Sotagliflozin and Gefitinib on the growth of colorectal cancer cell line HCT116;
Figure 2-f-1 shows the inhibition rate of different concentrations of Gefitinib alone on the growth of ovarian cancer cell line SKOV3; Figure 2-f-2 shows the inhibition rate of different concentrations of Sotagliflozin on ovarian cancer cell line SKOV3; Figure 2-f-3 shows the inhibition rate of 10 µmol/L Sotagliflozin with different concentrations of Gefitinib on the growth of ovarian cancer cell line SKOV3; Figure 2-f-4 shows the inhibition rate of 20 µmol/L Sotagliflozin with different concentrations of Gefitinib on the growth of ovarian cancer cell line SKOV3; Figure 2-f-5 shows the inhibition rate of 30 µmol/L Sotagliflozin with different concentrations of Gefitinib on the growth of ovarian cancer cell line SKOV3; and Figure 2-f-6 shows the inhibition rate of 40 µmol/L Sotagliflozin with different concentrations of Gefitinib on the growth of ovarian cancer cell line SKOV3;
Figure 2-g-1 shows the inhibition rate of different concentrations of Gefitinib alone on the growth of esophageal cancer cell line KYSE30; Figure 2-g-2 shows the inhibition rate of different concentrations of Sotagliflozin on esophageal cancer cell line KYSE30; Figure 2-g-3 shows the inhibition rate of 10 µmol/L Sotagliflozin with different concentrations of Gefitinib on the growth of esophageal cancer cell line KYSE30; Figure 2-g-4 shows the inhibition rate of 20 µmol/L Sotagliflozin with different concentrations of Gefitinib on the growth of esophageal cancer cell line KYSE30; Figure 2-g-5 shows the inhibition rate of 30 µmol/L Sotagliflozin with different concentrations of Gefitinib on the growth of esophageal cancer cell line KYSE30; and Figure 2-g-6 shows the inhibition rate of 40 µmol/L Sotagliflozin with different concentrations of Gefitinib on the growth of esophageal cancer cell line KYSE30;
Figure 2-h-1 shows the inhibition rate of different concentrations of Gefitinib alone on the growth of gastric cancer cell line HGC-27; Figure 2-h-2 shows the inhibitory effect of different concentrations of Sotagliflozin on gastric cancer cell line HGC-27; Figure 2-h-3 shows the inhibition rate of 10 µmol/L Sotagliflozin with different concentrations of Gefitinib on the growth of gastric cancer cell line HGC-27; Figure 2-h-4 shows the inhibition rate of 20 µmol/L Sotagliflozin with different concentrations of Gefitinib on the growth of gastric cancer cell line HGC-27; Figure 2-h-5 shows the inhibition rate of 30 µmol/L Sotagliflozin with different concentrations of Gefitinib on the growth of gastric cancer cell line HGC-27; and Figure 2-h-6 shows the inhibition rate of 40 µmol/L Sotagliflozin with different concentrations of Gefitinib on the growth of gastric cancer cell line HGC-27;
Figure 2-i-1 shows the inhibition rate of different concentrations of Gefitinib alone on the growth of cervical cancer cell line HeLa; Figure 2-i-2 shows the inhibitory effect of different concentrations of Sotagliflozin on cervical cancer cell line HeLa; Figure 2-i-3 shows the inhibition rate of 10 µmol/L Sotagliflozin with different concentrations of Gefitinib on the growth of cervical cancer cell line HeLa; Figure 2-i-4 shows the inhibition rate of 20 µmol/L Sotagliflozin with different concentrations of Gefitinib on the growth of cervical cancer cell line HeLa; Figure 2-i-5 shows the inhibition rate of 30 µmol/L Sotagliflozin with different concentrations of Gefitinib on the growth of cervical cancer cell line HeLa; and Figure 2-i-6 shows the inhibition rate of 40 µmol/L Sotagliflozin with different concentrations of Gefitinib on the growth of cervical cancer cell line HeLa;
Figure 2-j-1 shows the inhibition rate of different concentrations of Gefitinib alone on the growth of cholangiocarcinoma cell line RBE; Figure 2-j-2 shows the inhibitory effect of different concentrations of Sotagliflozin on cholangiocarcinoma cell line RBE; Figure 2-j-3 shows the inhibition rate of 10 µmol/L Sotagliflozin with different concentrations of Gefitinib on the growth of cholangiocarcinoma cell line RBE; Figure 2-j-4 shows the inhibition rate of 20 µmol/L Sotagliflozin with different concentrations of Gefitinib on the growth of cholangiocarcinoma cell line RBE; Figure 2-j-5 shows the inhibition rate of 30 µmol/L Sotagliflozin with different concentrations of Gefitinib on the growth of cholangiocarcinoma cell line RBE; and Figure 2-j-6 shows the inhibition rate of 40 µmol/L Sotagliflozin with different concentrations of Gefitinib on the growth of cholangiocarcinoma cell line RBE;
Figure 2-k-1 shows the inhibitory effect of different concentrations of Afatinib on lung cancer cell line A549; Figure 2-k-2 shows the inhibitory effect of different concentrations of Sotagliflozin on lung cancer cell line A549; Figure 2-k-3 shows the inhibitory effect of different concentrations of Afatinib +10 µM Sotagliflozin on lung cancer cell line A549 in test group 1; Figure 2-k-4 shows the inhibitory effect of different concentrations of Afatinib +20 µM Sotagliflozin on lung cancer cell line A549 in test group 2; Figure 2-k-5 shows the inhibitory effect of different concentrations of Afatinib +30 µM Sotagliflozin on lung cancer cell line A549 in test group 3; and Figure 2-k-6 shows the inhibitory effect of different concentrations of Afatinib +40 µM Sotagliflozin on lung cancer cell line A549 in test group 4;
Figure 2-l-1 shows the inhibitory effect of different concentrations of Erlotinib on lung cancer cell line A549; Figure 2-l-2 shows the inhibitory effect of different concentrations of Sotagliflozin on lung cancer cell line A549; Figure 2-l-3 shows the inhibitory effect of different concentrations of Erlotinib +10 µM Sotagliflozin on lung cancer cell line A549 in test group 1; Figure 2-l-4 shows the inhibitory effect of different concentrations of Erlotinib +20 µM Sotagliflozin on lung cancer cell line A549 in test group 2; Figure 2-l-5 shows the inhibitory effect of different concentrations of Erlotinib +30 µM Sotagliflozin on lung cancer cell line A549 in test group 3; and Figure 2-l-6 shows the inhibitory effect of different concentrations of Erlotinib +40 µM Sotagliflozin on lung cancer cell line A549 in test group 4;
Figure 3 shows the killing effect of Gefitinib, Sotagliflozin and the combination thereof on the Gefitinib resistant cell line A549 obtained after screening;
Figure 4-a-1 shows the inhibition rate of Apatinib alone and the combination of Sotagliflozin and Apatinib on the growth of hepatoma cell line HepG2;

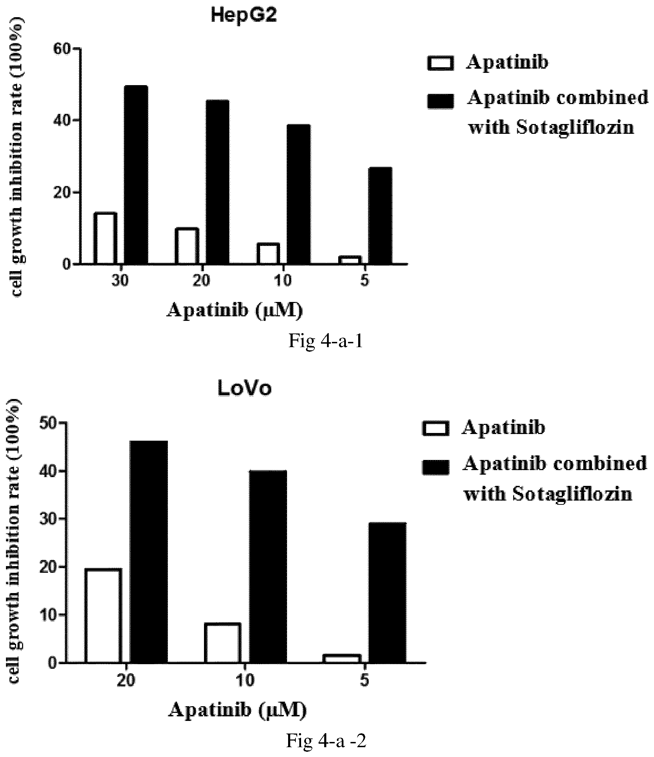

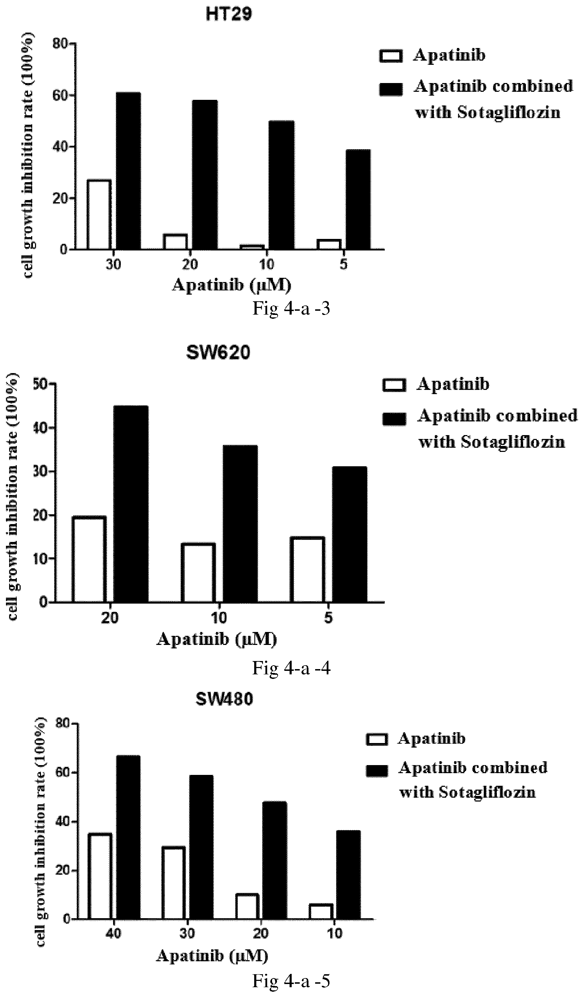

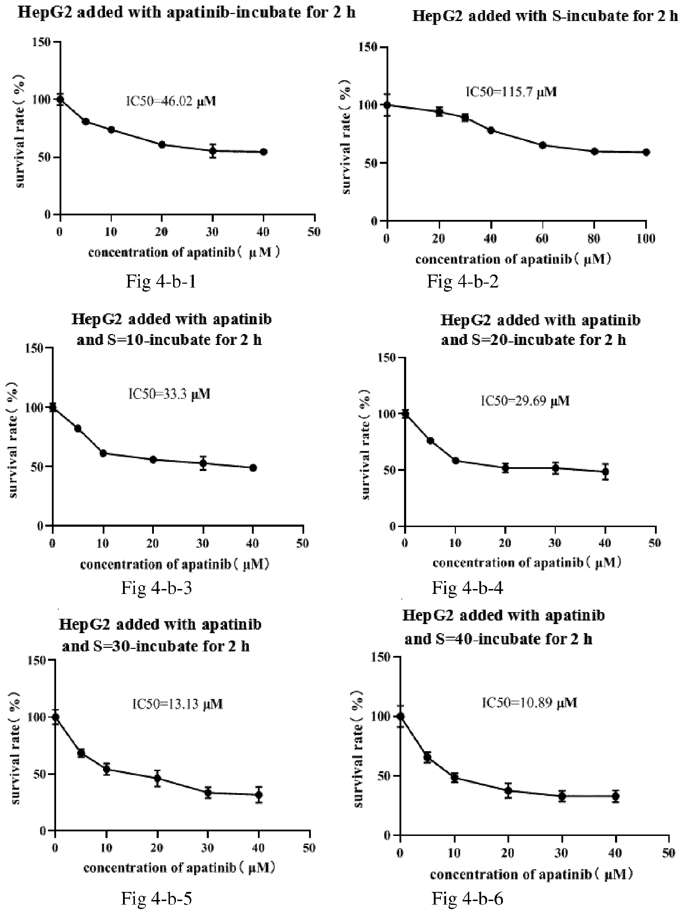

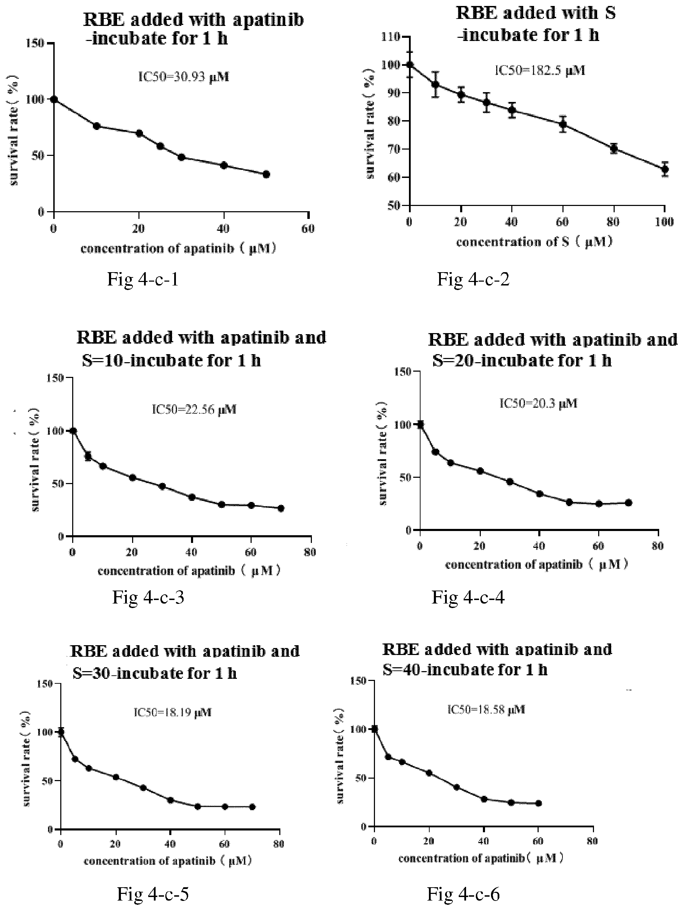

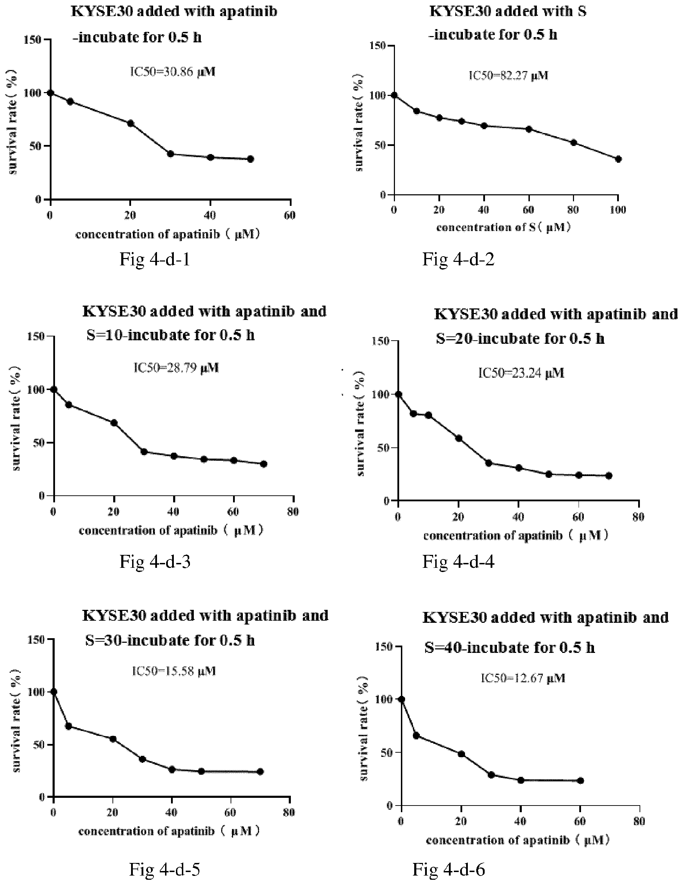

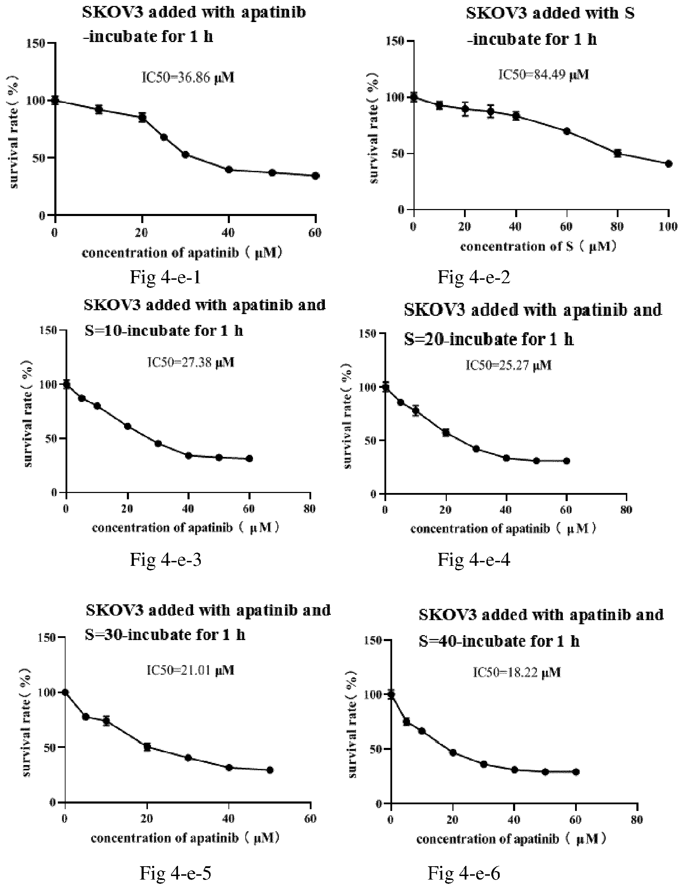

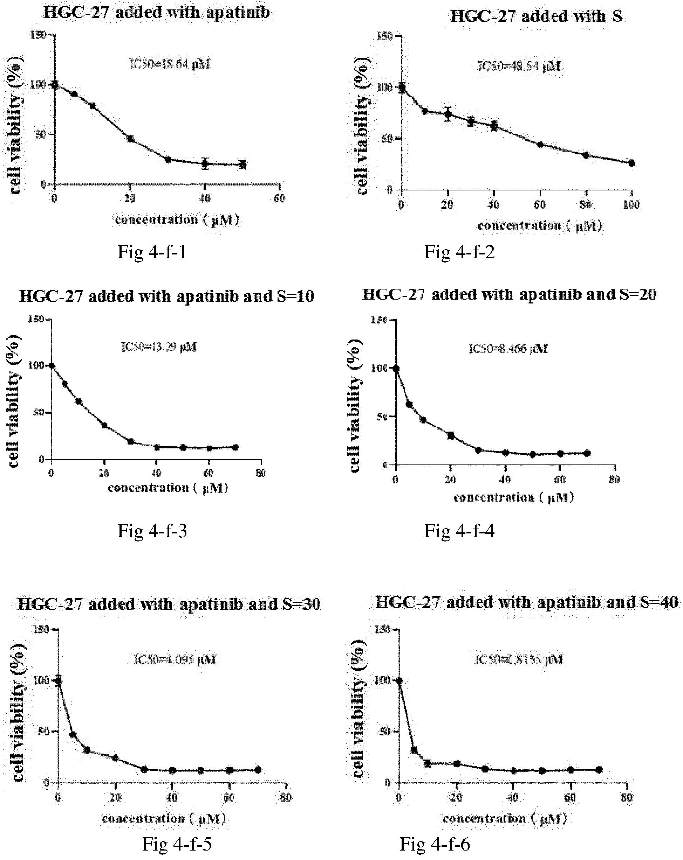

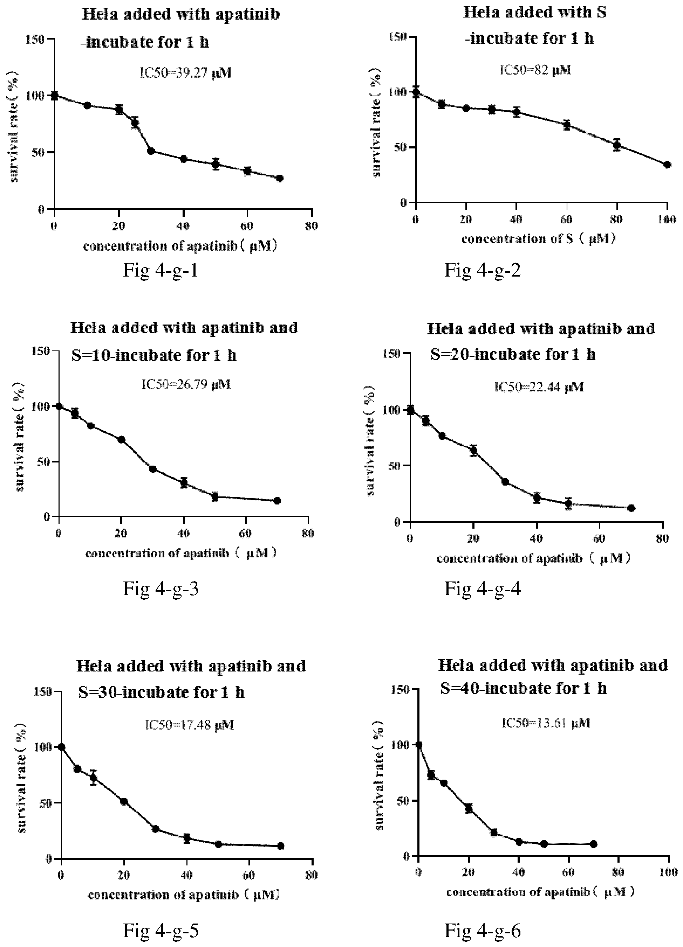

Figure 4-a-2 shows the inhibition rate of Apatinib alone and the combination of Sotagliflozin and Apatinib on the growth of colorectal cancer cell line LoVo;
Figure 4-a-3 shows the inhibition rate of Apatinib alone and the combination of Sotagliflozin and Apatinib on the growth of colorectal cancer cell line HT29;
Figure 4-a-4 shows the inhibition rate of Apatinib alone and the combination of Sotagliflozin and Apatinib on the growth of colorectal cancer cell line SW620;
Figure 4-a-5 shows the inhibition effect of Apatinib alone and the combination of Sotagliflozin and Apatinib on the growth of colorectal cancer cell line SW480;
Figure 4-b-1 shows the inhibitory effect of different concentrations of Apatinib on cell line HepG2;
Figure 4-b-2 shows the inhibitory effect of different concentrations of Sotagliflozin on cell line HepG2;
Figure 4-b-3 shows the inhibitory effect of different concentrations of Apatinib+ Sotagliflozin on cell line HepG2 in test group 1;
Figure 4-b-4 shows the inhibitory effect of different concentrations of Apatinib+ Sotagliflozin on cell line HepG2 in test group 2;
Figure 4-b-5 shows the inhibitory effect of different concentrations of Apatinib+ Sotagliflozin on cell line HepG2 in test group 3;
Figure 4-b-6 shows the inhibitory effect of different concentrations of Apatinib+ Sotagliflozin on cell line HepG2 in test group 4;
Figure 4-c-1 shows the inhibition rate of different concentrations of Apatinib alone on the growth of cholangiocarcinoma cell line RBE; Figure 4-c-2 shows the inhibition rate of different concentrations of Sotagliflozin alone on the growth of cholangiocarcinoma cell line RBE; Figure 4-c-3 shows the inhibition rate of 10 µmol/L Sotagliflozin with different concentrations of Apatinib on the growth of cholangiocarcinoma cell line RBE; Figure 4-c-4 shows the inhibition rate of 20 µmol/L Sotagliflozin with different concentrations of Apatinib on the growth of cholangiocarcinoma cell line RBE; Figure 4-c-5 shows the inhibition rate of 40 µmol/L Sotagliflozin with different concentrations of Apatinib on the growth of cholangiocarcinoma cell line RBE; Figure 4-c-6 shows the inhibition rate of 40 µmol/L Sotagliflozin with different concentrations of Apatinib on the growth of cholangiocarcinoma cell line RBE;
Figure 4-d-1 shows the inhibition rate of different concentrations of Apatinib alone on the growth of esophageal cancer cell line KYSE30; Figure 4-d-2 shows the inhibition rate of different concentrations of Sotagliflozin alone on the growth of esophageal cancer cell line KYSE30; Figure 4-d-3 shows the inhibition rate of 10 µmol/L Sotagliflozin with different concentrations of Apatinib on the growth of esophageal cancer cell line KYSE30; Figure 4-d-4 shows the inhibition rate of 20 µmol/L Sotagliflozin with different concentrations of Apatinib on the growth of esophageal cancer cell line KYSE30; Figure 4-d-5 shows the inhibition rate of 40 µmol/L Sotagliflozin with different concentrations of Apatinib on the growth of esophageal cancer cell line KYSE30; Figure 4-d-6 shows the inhibition rate of 40 µmol/L Sotagliflozin with different concentrations of Apatinib on the growth of esophageal cancer cell line KYSE30;
Figure 4-e-1 shows the inhibition rate of different concentrations of Apatinib alone on the growth of ovarian cancer cell line SKOV3; Figure 4-e-2 shows the inhibition rate of different concentrations of Sotagliflozin alone on the growth of ovarian cancer cell line SKOV3; Figure 4-e-3 shows the inhibition rate of 10 µmol/L Sotagliflozin with different concentrations of Apatinib on the growth of ovarian cancer cell line SKOV3; Figure 4-e-4 shows the inhibition rate of 20 µmol/L Sotagliflozin with different concentrations of Apatinib on the growth of ovarian cancer cell line SKOV3; Figure 4-e-5 shows the inhibition rate of 30 µmol/L Sotagliflozin with different concentrations of Apatinib on the growth of ovarian cancer cell line SKOV3; Figure 4-e-6 shows the inhibition rate of 40 µmol/L Sotagliflozin with different concentrations of Apatinib on the growth of ovarian cancer cell line SKOV3;
Figure 4-f-1 shows the inhibition rate of different concentrations of Apatinib alone on the growth of gastric cancer cell line HGC-27; Figure 4-f-2 shows the inhibition rate of different concentrations of Sotagliflozin alone on the growth of gastric cancer cell line HGC-27; Figure 4-f-3 shows the inhibition rate of 10 µmol/L Sotagliflozin with different concentrations of Apatinib on the growth of gastric cancer cell line HGC-27; Figure 4-f-4 shows the inhibition rate of 20 µmol/L Sotagliflozin with different concentrations of Apatinib on the growth of gastric cancer cell line HGC-27; Figure 4-f-5 shows the inhibition rate of 30 µmol/L Sotagliflozin with different concentrations of Apatinib on the growth of gastric cancer cell line HGC-27; Figure 4-f-6 shows the inhibition rate of 40 µmol/L Sotagliflozin with different concentrations of Apatinib on the growth of gastric cancer cell line HGC-27;
Figure 4-g-1 shows the inhibition rate of different concentrations of Apatinib alone on the growth of cervical cancer cell line HeLa; Figure 4-g-2 shows the inhibition rate of different concentrations of Sotagliflozin alone on the growth of cervical cancer cell line HeLa; Figure 4-g-3 shows the inhibition rate of 10 µmol/L Sotagliflozin with different concentrations of Apatinib on the growth of cervical cancer cell line HeLa; Figure 4-g-4 shows the inhibition rate of 20 µmol/L Sotagliflozin with different concentrations of Apatinib on the growth of cervical cancer cell line HeLa; Figure 4-g-5 shows the inhibition rate of 30 µmol/L Sotagliflozin with different concentrations of Apatinib on the growth of cervical cancer cell line HeLa; Figure 4-g-6 shows the inhibition rate of 40 µmol/L Sotagliflozin with different concentrations of Apatinib on the growth of cervical cancer cell line HeLa;

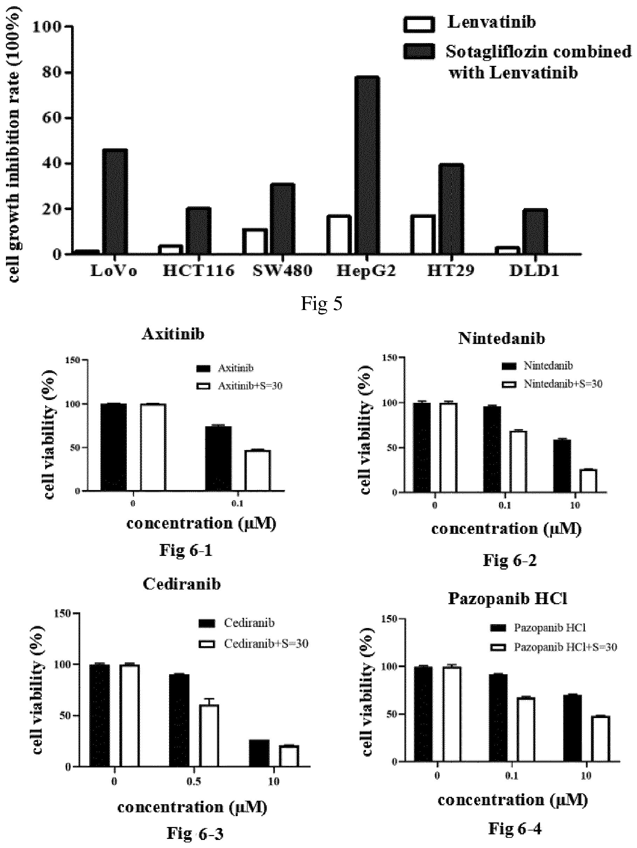

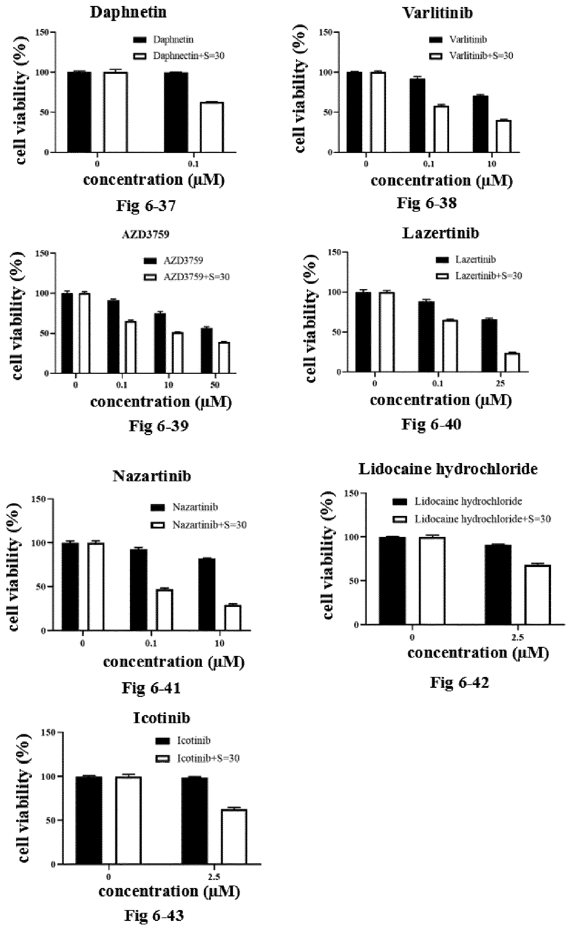

Figure 5 shows the inhibitory effect of Lenvatinib alone and the composition of Sotagliflozin combined with Lenvatinib on the growth of liver cancer HepG2 cells, colorectal cancer LoVo, HT29, DLD1, SW480, and HCT116 cells;

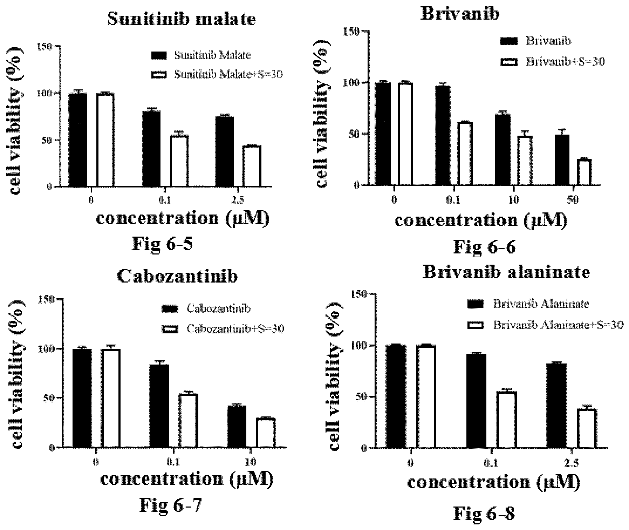

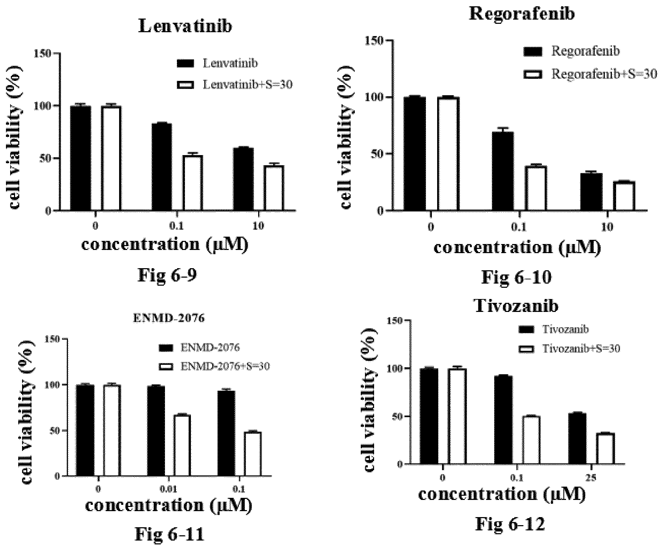

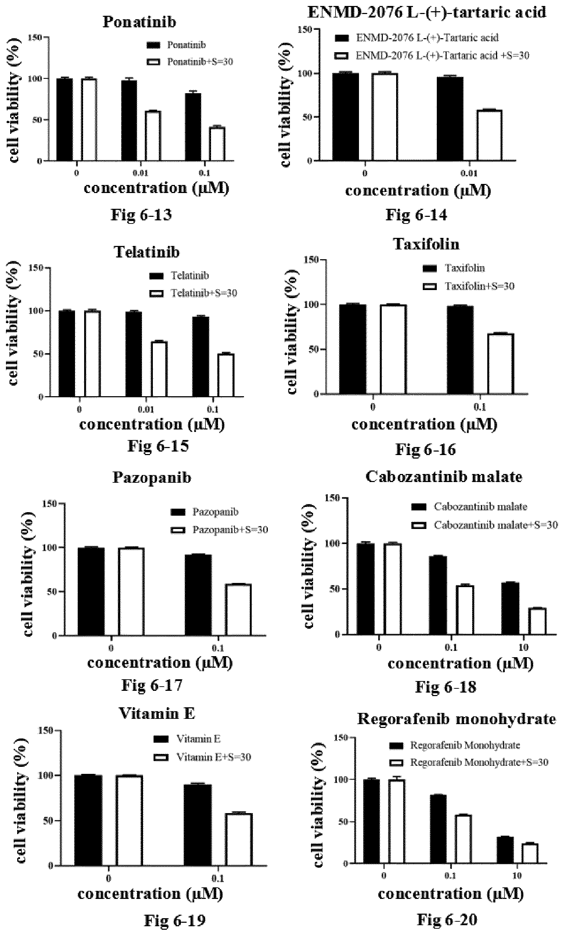

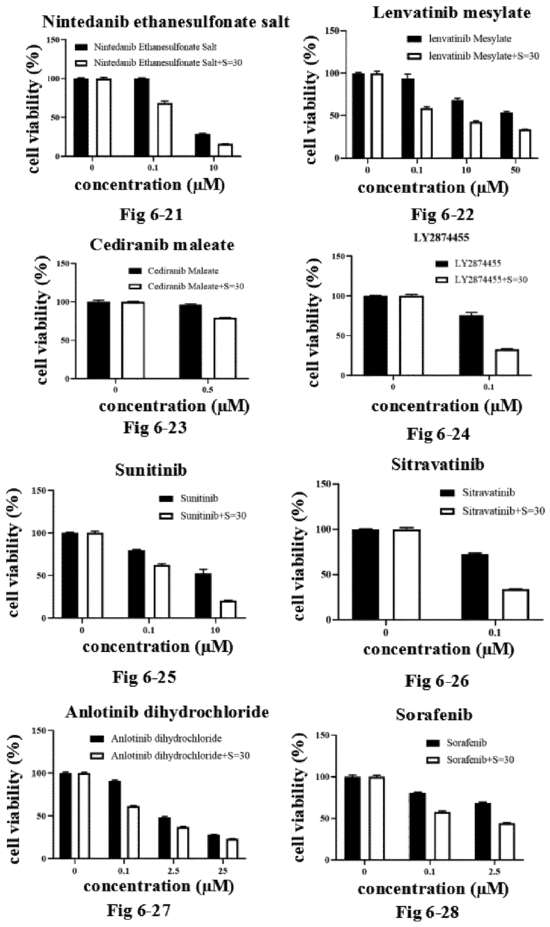

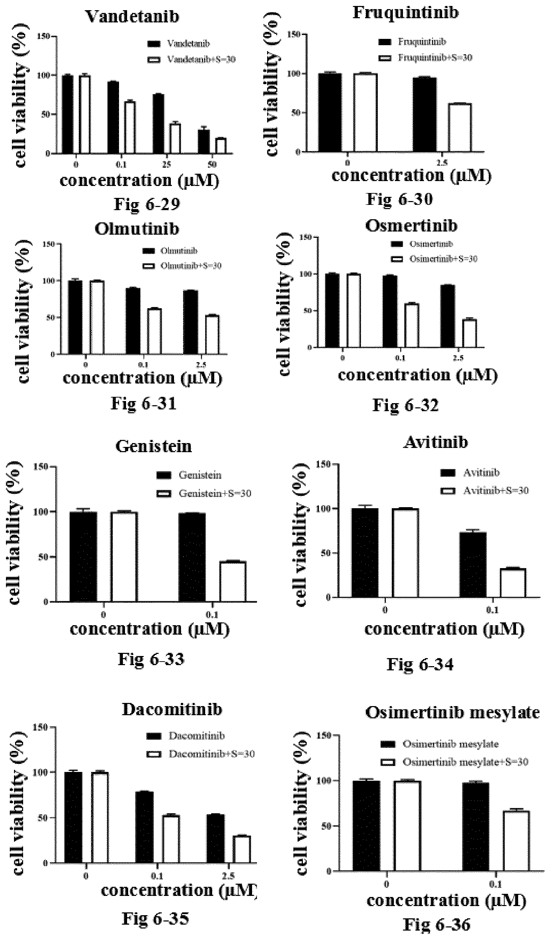

Figure 6-1 to Figure 6-43 show the effect of Sotagliflozin combined with Axitinib (Figure 6-1), Nintedanib (Figure 6-2), Cediranib (Figure 6-3), Pazopanib HCl (Figure 6-4), Sunitinib Malate (Figure 6-5), Brivanib (Figure 6-6), Cabozantinib (Figure 6-7), Brivanib Alaninate (Figure 6-8), Lenvatinib (Figure 6-9), Regorafenib (Figure 6-10), ENMD-2076 (Figure 6-11), Tivozanib (Figure 6-12), Ponatinib (Figure 6-13), ENMD-2076 L-(+)-Tartaric acid (Figure 6-14), Telatinib (Figure 6-15), Taxifolin (Figure 6-16), Pazopanib (Figure 6-17), Cabozantinib malate (Figure 6-18), Vitamin E (Figure 6-19), Regorafenib Monohydrate (Figure 6-20), Nintedanib Ethanesulfonate Salt (Figure 6-21), Lenvatinib Mesylate (Figure 6-22), Cediranib Maleate (Figure 6-23), LY2874455 (Figure 6-24), Sunitinib (Figure 6-25), Sitravatinib (Figure 6-26), Anlotinib (Figure 6-27), Sorafenib (Figure 6-28), Vandetanib (Figure 6-29), Fruquintinib (Figure 6-30), Olmutinib (Figure 6-31), Osimertinib (Figure 6-32), Genistein (Figure 6-33), Avitinib (Figure 6-34), Dacomitinib (6-35), Osimertinib mesylate (Figure 6-36), Daphnetin (Figure 6-37), Varlitinib (Figure 6-38), AZD3759 (Figure 6-39), Lazertinib (Figure 6-40), Nazartinib (Figure 6-41), Lidocaine Hydrochloride (Figure 6-42), and Icotinib (Figure 6-43), respectively;

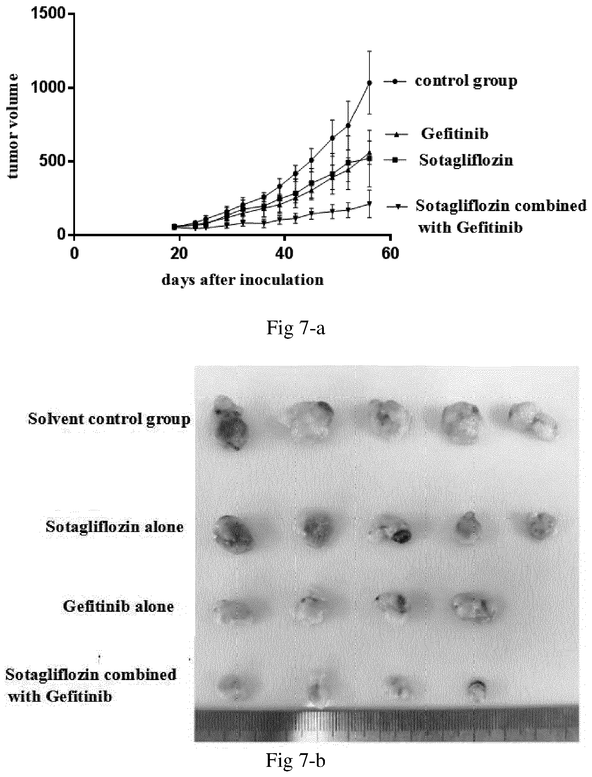

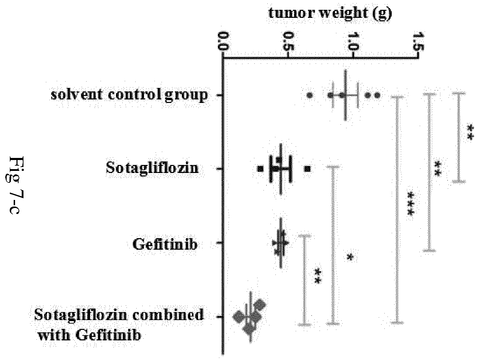

Figure 7-a shows the growth curve of tumor in the tumor-bearing mice modeled by lung cancer A549 cells during the administration of Sotagliflozin alone, Apatinib alone, and the combination of Sotagliflozin and Apatinib;
Figure 7-b shows the macroscopic view of tumor of the tumor-bearing mice modeled by lung cancer A549 cells after the administration of Sotagliflozin alone, Gefitinib alone, and the combination of Sotagliflozin and Gefitinib;
Figure 7-c shows the weight of mouse tumors after the administration of Sotagliflozin alone, Gefitinib alone and the combination of Sotagliflozin and Gefitinib.

### DETAILED DESCRIPTION

The present invention Sotagliflozin or a pharmaceutically acceptable salt thereof combined with Gefitinib for the use in treating lung cancer resistant to Gefitinib. The Sotagliflozin or a pharmaceutically acceptable salt thereof combined with Gefitinib for the use in treating lung cancer resistant to Gefitinib according to the present invention have been described through the preferred examples.

Unless specifically stated otherwise in the present disclosure, all technical and scientific terms involved in the present disclosure have the same meanings as commonly understood by those in the art. The technology used in the present disclosure is intended to refer to the technology generally understood in the art, including changes or equivalent replacements of the technology obvious to those skilled in the art. Although it is believed that the following terms are well understood by those skilled in the art, the following definitions are provided to better explain the present disclosure.

As used herein, the terms "including", "comprising", "having", "containing" or "involving" and other variants thereof herein are inclusive or open-ended, and do not exclude other non-listed elements or method steps

Therefore, the present disclosure relates to use of a dual inhibitor Sotagliflozin of SGLT1 and SGLT2, the two most important members of the SGLT family highly expressed in cancer cells, and a composition comprising TKI in the manufacture of a medicament for treating cancer.

The term "treatment" as used herein means that after administration of the medicament of the present disclosure, the experimental animal suffering from a disease or condition shows partial or full relief of the symptoms, or the symptoms do not continue to worsen after treatment. Therefore, treatment includes cure.

As used herein, "therapeutic effect" refers to the effect caused by the treatment, which is manifested as cell growth inhibition rate or cell death rate at the cellular level, and changes, generally reduction or improvement of symptoms of a disease or condition, or cure of the disease or condition at the animal level. In the present disclosure, a medicament is effective if the tumor growth inhibition rate is greater than 60%, and the p-value of the statistical difference of the tumor volume or weight between the treatment group and the control group is less than 0.05.

As used herein, "cell growth inhibition rate" refers to the ratio of the average value of the absorbance of the cells stained with MTT in the treatment group to the average value of the absorbance in the control group after drug treatment. "Tumor growth inhibition rate" represents the ratio of the average tumor volume or weight of the treatment group after drug treatment to the average volume or weight of the control group.

According to the present disclosure, the Sotagliflozin is used to treat cancer in a subject.

The term "cancer" as used herein refers to the malignant proliferation of epithelial cells due to changes in genetic material. The cancers include: bladder cancer, blood cancer, bone cancer, brain cancer, breast cancer, central nervous system cancer, cervical cancer, colon cancer, endometrial cancer, esophageal cancer, gallbladder cancer, gastrointestinal cancer, external genital cancer, urogenital cancer, head cancer, kidney cancer, laryngeal cancer, liver cancer, muscle tissue cancer, neck cancer, oral or nasal mucosal cancer, ovarian cancer, prostate cancer, skin cancer, spleen cancer, small bowel cancer, large bowel cancer, stomach cancer, testicular cancer and/or thyroid cancer.

According to the invention, the cancer is lung cancer resistant to Gefitinib.

The test materials used in the present disclosure are all common commercially available products, and all are available in the market.

In the examples of this application, the drugs involved and their Chinese names are shown in Table 1:

**Table 1 Drugs and their Chinese names**

| Chinese name | English name |
|---|---|
| 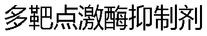 | ENMD-2076 |
| 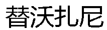 | Tivozanib |
| 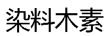 | Genistein |
| 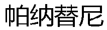 | Ponatinib |
| 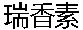 | Daphnetin |
| 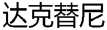 | Dacomitinib |
| 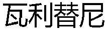 | Varlitinib |
| 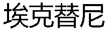 | Icotinib |
| 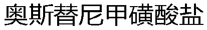 | Osimertinib mesylate |
| 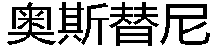 | Osimertinib |
| 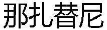 | Nazartinib |
| | AZD3759 |
| 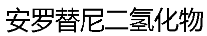 | Anlotinib (AL3818) dihydrochloride |
| 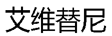 | Avitinib |
| 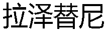 | Lazertinib |
| 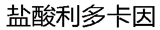 | Lidocaine hydrochloride |
| 4-[(1E)-2-[5-[(1R)-1-(3,5- 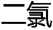 -4- 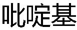 ) 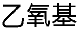 ]-1H- 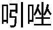 -3- 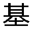 ] 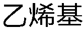 ]-1H- 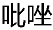 -1- 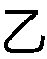 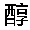 | LY2874455 |
| 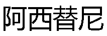 | Axitinib |
| 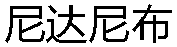 | Nintedanib |
| 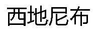 | Cediranib |
| 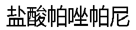 | Pazopanib HCl |
| 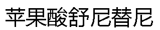 | Sunitinib Malate |
| 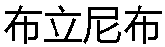 | Brivanib |
| 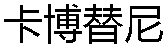 | Cabozantinib |
| 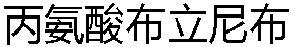 | Brivanib Alaninate |
| 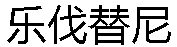 | Lenvatinib |
| 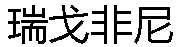 | Regorafenib |
| ENMD-2076 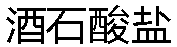 | ENMD-2076 L-(+)-Tartaric acid |
| 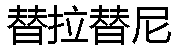 | Telatinib |
| 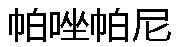 | Pazopanib |
| 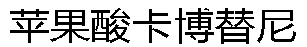 | Cabozantinib malate |
| 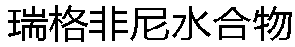 | Regorafenib Monohydrate |
| 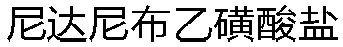 | Nintedanib Ethanesulfonate Salt |
| 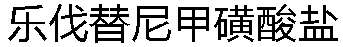 | Lenvatinib Mesylate |
| 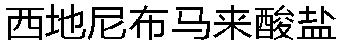 | Cediranib Maleate |
| 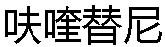 | Fruquintinib |
| 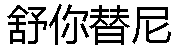 | Sunitinib |
| 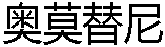 | Olmutinib |
| 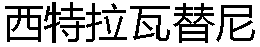 | Sitravatinib |
| 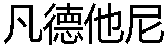 | Vandetanib |
| 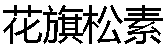 | Taxifolin (Dihydroquercetin) |
| 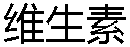 E | Vitamin E |
| 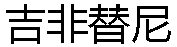 | Gefitinib |
| 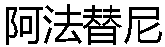 | Afatinib |
| 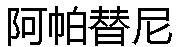 | Apatinib |
| 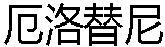 | Erlotinib |
| 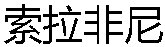 | Sorafenib |
| 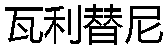 | Varlitinib |

the present disclosure will be now further explained with respect to the examples:

### Reference

### Example 1 The inhibitory effect of Sotagliflozin on tumor cells

Prostate cancer DU145 cells, breast cancer MCF-7 cells, esophageal cancer KYSE30 cells, gastric cancer HGC-27 cells, cholangiocarcinoma RBE cells, ovarian cancer SKOV3 cells, and cervical cancer Hela cells were used to verify the inhibitory effect of Sotagliflozin on tumor cells. After growing to 80% density, the cells were trypsinized, passaged and plated in a 96-well plate with 5000 cells per well. After 24 hours, the medium was replaced with a medium containing the corresponding concentration of drug. After 48 hours, the absorbance at each concentration was detected by the MTT method.

The experiment included the following groups:
Control group: cells were cultured in normal culture medium without drug added.
Sotagliflozin test group: cells were treated by adding Sotagliflozin at different concentration to each culture medium.

After incubation, the cell growth inhibition rate was calculated by dividing the absorbance value at each concentration by the absorbance value of the control group. The calculated IC₅₀ values of Sotagliflozin on various tumor cells are shown in Figure 1-a to Figure 1-g. In the figures, the concentration of Sotagliflozin is taken as the abscissa and the cell growth inhibition rate is taken as the ordinate. The results show that Sotagliflozin has a certain inhibitory effect on various tumor cells.

### Example 2 Sotagliflozin combined with Gefitinib

### Determination of the safe concentration of Sotagliflozin

Sotagliflozin was purchased from Selleck Chemicals for growth inhibition test of cancer cell *in vitro.* Initially, test using normal human umbilical cord epithelial cells showed that Sotagliflozin produced great cytotoxicity at a concentration higher than 80 µM, and mainly exhibited cell growth inhibition effect at a concentration lower than 80µM. Therefore, the concentrations of Sotagliflozin used in the subsequent compositions of this experiment were all lower than 80 µM to avoid affecting normal cells.

### 2. The inhibitory effect of combination administration on tumor cells

According to the tissue distribution characteristics of EGFR and SGLT1/2, the targets of Gefitinib and Sotagliflozin, the followings cells were selected for experimental verification: lung cancer cell line A549; colorectal cancer cell line LoVo, HT29, SW620, HCT116; cervical cancer HeLa; ovarian cancer SKOV3; gastric cancer HGC27; cholangiocarcinoma RBE; esophageal cancer KYSE30, and the like. After growing to 80% density, the cells were trypsinized, passaged and plated in a 96-well plate with 5000 cells per well. After 24 hours, the medium was replaced with a medium containing the corresponding concentration of drug. After 48 hours, the absorbance at each concentration was detected by the MTT method.

The experiment included the following groups:
Control group: cells were cultured in normal culture medium without drug added.
Gefitinib test group: cells were treated by adding Gefitinib alone at 4 different concentration, 5µM, 10µM, 20µM, 30µM, respectively, to the culture medium.
Gefitinib + Sotagliflozin combination test group: cells were treated by adding both Sotagliflozin (20 µM) and Gefitinib at 4 different concentration, 5µM, 10µM, 20µM and 30µM, respectively, to the culture medium.

After incubation, the cell growth inhibition rate was calculated by dividing the absorbance value at each concentration by the absorbance value of the control group. The results are shown in Figure 2-a to Figure 2-e. In the figures, the concentration of Gefitinib is taken as the abscissa and the cell growth inhibition rate is taken as the ordinate. The results showed that the inhibitory effect of Gefitinib alone on tumor cells is limited, and the combination of the two drugs is beneficial to improve the tumor inhibitory effect.

### Determination of IC₅₀ value of combination administration

Lung cancer cell line A549 was used as an example to verify the IC₅₀ value of the Gefitinib + Sotagliflozin combination. The experiment included the following groups:
Gefitinib test group (Figure 2-a-1): cells were treated by adding Gefitinib alone at 6 different concentrations, 0µM, 10µM, 20µM, 30µM, 40µM and 50µM respectively, to the culture medium, and cell survival rate was measured after 1h of incubation. The results showed that the IC₅₀ value of Gefitinib on A549 cells was 24.42µM.
Sotagliflozin test group (Figure 2-a-2): cells were treated by adding Sotagliflozin alone at 6 different concentrations, 0µM, 10µM, 30µM, 40µM, 50µM and 60µM respectively, to the culture medium, and cell survival rate was measured after 1h of incubation. The results showed that the IC₅₀ value of Sotagliflozin on A549 cells was 73.04µM.
Gefitinib + Sotagliflozin combination test group 1 (Figure 2-a-3): cells were treated by adding both Sotagliflozin (10 µM) and Gefitinib at 6 different concentrations, 0µM, 10µM, 20µM, 30µM, 50µM and 60µM respectively, to the culture medium, and cell survival rate was measured after 1h of incubation. The results showed that the IC₅₀ value of the test group to on A549 cells was 17.03 µM.
Gefitinib + Sotagliflozin combination test group 2 (Figure 2-a-4): cells were treated by adding both Sotagliflozin (20 µM) and Gefitinib at 5 different concentrations, 0µM, 10µM, 20µM, 30µM and 40µM, respectively, to the culture medium, and cell survival rate was measured after 2h of incubation. The results showed that the IC₅₀ value of the test group to on A549 cells was 12.71 µM.
Gefitinib + Sotagliflozin combination test group 3 (Figure 2-a-5): cells were treated by adding both Sotagliflozin (30 µM) and Gefitinib at 6 different concentrations, 0µM, 10µM, 20µM, 30µM, 40µM and 50µM respectively, to the culture medium, and cell survival rate was measured after 1h of incubation. The results showed that the IC₅₀ value of the test group to on A549 cells was 9.318 µM.

The results showed that with the combination of Sotagliflozin and Gefitinib, the inhibitory effect of Gefitinib on A549 cells was significantly enhanced, and the IC₅₀ was reduced to less than half of that of the single agent. Therefore, the effect of the combination in the safe concentration range of Sotagliflozin is better than the effect of each single agent. The verification results of other cell lines are shown in the figures (2f-2j). It is worth mentioning that the ability of Sotagliflozin in the combination to enhance the efficacy of EGFR-targeting TKI drugs is not limited to a single drug of Gefitinib. In Figure 2k and Figure 2l, the present disclosure takes A549 cells as an example to further verify the other two inhibitors of EGFR, Afatinib and Erlotinib, and the results showed that the addition of Sotagliflozin at an a safe dose can effectively reduce a IC₅₀ values of Afatinib and Erlotinib.

### Example 3 Sotagliflozin combined with Gefitinib reverses the resistance of tumor cells to Gefitinib

### Screening of Gefitinib-resistant cell lines.

After knowing that the combination of Gefitinib and Sotagliflozin significantly enhanced the effectiveness of Gefitinib from example 1, the present disclosure continues to explore whether the combination of the two drugs can reverse the resistance of tumor cells to Gefitinib. Gefitinib-resistant cells can be obtained by long-term culturing of A549 cells with a medium containing Gefitinib at increasing concentrations. After 5 months of screening, the present disclosure obtained A549 gefitinib-resistant cell line that can survive in 60 µM gefitinib for a long time.

### 2. Sotagliflozin combined with Gefitinib reverses the resistance of tumor cells to Gefitinib

The Gefitinib-resistant cell line obtained by the present disclosure can be effectively killed by adding 30 µM Gefitinib and Sotagliflozin composition. It shows that the combination of Sotagliflozin and Gefitinib reverses the resistance of tumor cells to Gefitinib. The results are shown in Figure 3.

### Example 4 Sotagliflozin combined with Apatinib (example not falling within the scope of the present invention)

The inhibitory effect of combination administration on tumor cells

After obtaining the effectiveness of Gefitinib in Example 1, the present disclosure further validated another VEGFR-targeting TKI drug, Apatinib. According to the tissue distribution characteristics of VEGFR and SGLT1/2, the targets of Apatinib and Sotagliflozin, the followings cells were selected for experimental verification: liver cancer cell line HepG2; colorectal cancer cell line LoVo, HT29, SW620, SW480; cervical cancer HeLa; ovarian cancer SKOV3; gastric cancer HGC27; cholangiocarcinoma RBE; esophageal cancer KYSE3, and the like. After growing to 80% density, the cells were trypsinized, passaged and plated in a 96-well plate with 5000 cells per well. After 24 hours, the medium was replaced with a medium containing the corresponding concentration of Apatinib, Sotagliflozin, or the combination of Apatinib and Sotagliflozin. After 48 hours, the absorbance at each concentration was detected by the MTT method.

The experiment included the following groups:
Control group: cells were cultured in normal culture medium without drug added.
Apatinib test group: cells were treated by adding Apatinib alone at 4 different concentrations, 5µM, 10µM, 20µM, 30µM, respectively, to the culture medium.
Apatinib + Sotagliflozin combination test group: cells were treated by adding both Sotagliflozin (20 µM) and Apatinib at 4 different concentrations, 5µM, 10µM, 20µM and 30µM, respectively, to the culture medium.

The cell growth inhibition rate was calculated by dividing the absorbance value at each concentration by the absorbance value of the control group. The results are shown in Figure 4-a-1 to Figure 4-a-5. In the figures, the concentration of Apatinib is taken as the abscissa and the cell growth inhibition rate is taken as the ordinate. The results show that the inhibitory effect of Apatinib alone on tumor cells is limited, and the combination of the two drugs is beneficial to improve the tumor inhibitory effect.

### 2. Determination of IC₅₀ value of combination administration

### 2.1. Liver cancer cell line HepG2 was used as an example to verify the IC₅₀ value of the Apatinib + Sotagliflozin combination. The experiment included the following groups:

Apatinib test group (Figure 4-b-1): cells were treated by adding Apatinib alone at 6 different concentrations, 0µM, 5µM, 10µM, 20µM, 30µM and 40µM, respectively, to the culture medium, and cell survival rate was measured after 2h of incubation. The results showed that the IC₅₀ value of Apatinib on HepG2 cells was 46.02µM.

Sotagliflozin test group (Figure 4-b-2): cells were treated by adding Sotagliflozin alone at 7 different concentrations, 0µM, 20µM, 30µM, 40µM, 60µM, 80µM and 100µM respectively, to the culture medium, and cell survival rate was measured after 2h of incubation. The results showed that the IC₅₀ value of Sotagliflozin on HepG2 cells was 115.7µM.

Apatinib + Sotagliflozin combination test group 1 (Figure 4-b-3): cells were treated by adding both Sotagliflozin (10 µM) and Apatinib at 6 different concentrations, 0µM, 5µM, 10µM, 20µM, 30µM and 40µM respectively, to the culture medium, and cell survival rate was measured after 2h of incubation. The results showed that the IC₅₀ value of the test group to on HepG2 cells was 33.3 µM.

Apatinib + Sotagliflozin combination test group 2 (Figure 4-b-4): cells were treated by adding both Sotagliflozin (20 µM) and Apatinib at 6 different concentrations, 0µM, 5µM, 10µM, 20µM, 30µM and 40µM respectively, to the culture medium, and cell survival rate was measured after 2h of incubation. The results showed that the IC₅₀ value of the test group to on HepG2 cells was 29.69µM.

Apatinib + Sotagliflozin combination test group 3 (Figure 4-b-5): cells were treated by adding both Sotagliflozin (30 µM) and Apatinib at 6 different concentrations, 0µM, 5µM, 10µM, 20µM, 30µM and 40µM respectively, to the culture medium, and cell survival rate was measured after 2h of incubation. The results showed that the IC₅₀ value of the test group to on HepG2 cells was 13.13 µM.

Apatinib + Sotagliflozin combination test group 4 (Figure 4-b-6): cells were treated by adding both Sotagliflozin (40 µM) and Apatinib at 6 different concentrations, 0µM, 5µM, 10µM, 20µM, 30µM and 40µM respectively, to the culture medium, and cell survival rate was measured after 2h of incubation. The results showed that the IC₅₀ value of the test group to on HepG2 cells was 10.89 µM.

These results showed that with the combination of Sotagliflozin and Apatinib, the inhibitory effect of Apatinib on HepG2 cells was significantly enhanced, and the IC₅₀ was reduced to less than one-fourth of that of the single agent. Therefore, the effect of the combination in the safe concentration range of Sotagliflozin is better than the effect of each single agent. The verification results of other cell lines are shown in the figures (4c-4g). It is worth mentioning that the ability of Sotagliflozin in the combination to enhance the efficacy of VEGFR-targeting TKI drugs is not limited to a single drug of Apatinib. In Figure 5, the present disclosure further verified another VEGFR inhibitor

Lenvatinib in a variety of cell lines, and the results showed that the addition of Sotagliflozin at a safe dose enhanced the inhibitory effect of Lenvatinib on these cell lines.

### Example 5 (example not falling within the scope of the present invention)

Other TKI drugs were further verified, proving that the TKI drugs which can be combined with Sotagliflozin are not limited to specific one or several drugs.

The selected drugs included: Axitinib (Figure 6-1), Nintedanib (Figure 6-2), Cediranib (Figure 6-3), Pazopanib HCl (Figure 6-4), Sunitinib Malate (Figure 6-5), Brivanib (Figure 6-6), Cabozantinib (Figure 6-7), Brivanib Alaninate (Figure 6-8), Lenvatinib (Figure 6-9), Regorafenib (Figure 6-10), ENMD-2076 (Figure 6-11), Tivozanib (Figure 6-12), Ponatinib (Figure 6-13), ENMD-2076 L-(+)-Tartaric acid (Figure 6-14), Telatinib (Figure 6-15), Taxifolin (Figure 6-16), Pazopanib (Figure 6-17), Cabozantinib malate (Figure 6-18), Vitamin E (Figure 6-19), Regorafenib Monohydrate (Figure 6-20), Nintedanib Ethanesulfonate Salt (Figure 6-21), Lenvatinib Mesylate (Figure 6-22), Cediranib Maleate (Figure 6-23), LY2874455 (Figure 6-24), Sunitinib (Figure 6-25), Sitravatinib (Figure 6-26), Anlotinib (Figure 6-27), Sorafenib (Figure 6-28), Vandetanib (Figure 6-29), Fruquintinib (Figure 6-30), Olmutinib (Figure 6-31), Osimertinib (Figure 6-32), Genistein (Figure 6-33), Avitinib (Figure 6-34), Dacomitinib (Figure 6-35), Osimertinib mesylate (Figure 6-36), Daphnetin (Figure 6-37), Varlitinib (Figure 6-38), AZD3759 (Figure 6-39), Lazertinib (Figure 6-40), Nazartinib (Figure 6-41), Lidocaine Hydrochloride (Figure 6-42), icotinib (Figure 6-43)
the present disclosure preferentially selected liver cancer cell line HepG2 for experimental verification. After growing to 80% density, the cells were trypsinized, passaged and plated in a 96-well plate with 5000 cells per well. After 24 hours, the medium was replaced with a medium containing the corresponding concentration of Apatinib, Sotagliflozin, or the combination of Apatinib and Sotagliflozin. After 48 hours, the absorbance at each concentration was detected by the MTT method.

The experimental method was the same as before, and the incubation time was 1h. The experiment included a blank control group, namely normal cultured HepG2 cells, in which the concentrations of Sotagliflozin or TKI drugs were both 0, and the survival rate of the blank control group cells was set to 100%. In other groups, the concentration of Sotagliflozin used was 30 µmol/L, and the concentrations of TKI drugs are shown in Table 2. The results are shown in the attached Figures:

**Table 2 Experimental design of the administration group**

| Group | Group 1 | Group 2 | Group 3 | Group 4 |
|---|---|---|---|---|
| Axitinib | Sotagliflozin 30µmol/L + Axitinib 0 | Sotagliflozin 30µmol/L + Axitinib 0.1µmol/L | -- | -- |
| Brivanib Alaninate | Sotagliflozin 30µmol/L + Brivanib Alaninate 0 | Sotagliflozin 30µmol/L + Brivanib alaninate 0.1µmol/L | Sotagliflozin 30µmol/L + Brivanib alaninate 2.5µmol/L | -- |
| Pazopanib | Sotagliflozin 30µmol/L + Pazopanib 0 | Sotagliflozin 30µmol/L + Pazopanib 0.1µmol/L | -- | -- |
| Cediranib Maleate | Sotagliflozin 30µmol/L + Cediranib Maleate 0 | Sotagliflozin 30µmol/L + Cediranib maleate 0.5µmol/L | -- | -- |
| Olmutinib | Sotagliflozin 30µmol/L + Olmutinib 0 | Sotagliflozin 30µmol/L + Olmutinib 0.1µmol/L | Sotagliflozin 30µmol/L + Olmutinib 2.5µmol/L | -- |
| Nintedanib | Sotagliflozin 30µmol/L + Nintedanib 0 | Sotagliflozin 30µmol/L + Nintedanib 0.1µmol/L | Sotagliflozin 30µmol/L + Nintedanib 10µmol/L | -- |
| Lenvatinib | Sotagliflozin 30µmol/L + Lenvatinib 0 | Sotagliflozin 30µmol/L + Lenvatinib 0.1 µmol/L | Sotagliflozin 30µmol/L + Lenvatinib 10µmol/L | -- |
| Cabozantinib malate | Sotagliflozin 30µmol/L + Cabozantinib malate 0 | Sotagliflozin 30µmol/L + Cabozantinib malate 0.1µmol/L | Sotagliflozin 30µmol/L + Cabozantinib malate 10µmol/L | -- |
| Sorafenib | Sotagliflozin 30µmol/L + Sorafenib0 | Sotagliflozin 30µmol/L + Sorafenib0.1 µmol/L | Sotagliflozin 30µmol/L + Sorafenib10µmol/L | -- |
| Cediranib | Sotagliflozin 30µmol/L + Cediranib 0 | Sotagliflozin 30µmol/L + Cediranib 0.5µmol/L | Sotagliflozin 30µmol/L + Cediranib 10µmol/L | -- |
| Regorafenib | Sotagliflozin 30µmol/L + Regorafenib 0 | Sotagliflozin 30µmol/L + Regorafenib 0.1µmol/L | Sotagliflozin 30µmol/L + Regorafenib 10µmol/L | -- |
| Telatinib | Sotagliflozin 30µmol/L + Telatinib 0 | Sotagliflozin 30µmol/L + Telatinib 0.1µmol/L | Sotagliflozin 30µmol/L + Telatinib 10µmol/L | -- |
| Lidocaine hydrochloride | Sotagliflozin 30µmol/L + Lidocaine hydrochloride 0 | Sotagliflozin 30µmol/L + Lidocaine hydrochloride 2.5 µmol/L | -- | -- |
| LY2874455 | Sotagliflozin 30µmol/L + LY2874455 0 | Sotagliflozin 30µmol/L + LY2874455 0.1 µmol/L | -- | -- |
| Sitravatinib | Sotagliflozin 30µmol/L + Sitravatinib 0 | Sotagliflozin 30µmol/L + Sitravatinib 0.1 µmol/L | -- | -- |
| ENMD-2076 | Sotagliflozin 30µmol/L + ENMD-2076 0 | Sotagliflozin 30µmol/L + ENMD-20760.1 µmol/L | -- | -- |
| Taxifolin | Sotagliflozin 30µmol/L + Taxifolin0 | Sotagliflozin 30µmol/L + Taxifolin0.1 µmol/L | -- | -- |
| Vitamin E | Sotagliflozin 30µmol/L + Vitamin E 0 | Sotagliflozin 30µmol/L + Vitamin E 0.1 µmol/L | -- | -- |
| Osimertinib | Sotagliflozin 30µmol/L + Osimertinib 0 | Sotagliflozin 30µmol/L + Osimertinib 0.1 µmol/L | Sotagliflozin 30µmol/L + Osimertinib 2.5 µmol/L | -- |
| Anlotinib dihydrochlo | Sotagliflozin 30µmol/L + | Sotagliflozin 30µmol/L + Anlotinib | Sotagliflozin 30µmol/L + | Sotagliflozin 30µmol/L + |
| ride | Anlotinib dihydrochloride 0 | dihydrochloride 0.1 µmol/L | Anlotinib dihydrochloride 2.5 µmol/L | Anlotinib dihydrochloride 25 µmol/L |
| Pazopanib HCl | Sotagliflozin 30µmol/L + Pazopanib HCl 0 | Sotagliflozin 30µmol/L + Pazopanib HCl 0.1 µmol/L | Sotagliflozin 30µmol/L + Pazopanib HCl 10 µmol/L | -- |
| Tivozanib | Sotagliflozin 30µmol/L + Tivozanib 0 | Sotagliflozin 30µmol/L + Tivozanib 0.1 µmol/L | Sotagliflozin 30µmol/L + Tivozanib 25 µmol/L | -- |
| Regorafenib Monohydrate | Sotagliflozin 30µmol/L + Regorafenib Monohydrate0 | Sotagliflozin 30µmol/L + Regorafenib monohydrate 0.1 µmol/L | Sotagliflozin 30µmol/L + Regorafenib monohydrate 10 µmol/L | -- |
| Avitinib | Sotagliflozin 30µmol/L + Avitinib 0 | Sotagliflozin 30µmol/L + Avitinib 0.1 µmol/L | -- | -- |
| Sunitinib Malate | Sotagliflozin 30µmol/L + Sunitinib Malate0 | Sotagliflozin 30µmol/L + Sunitinib Malate0.1 µmol/L | Sotagliflozin 30µmol/L + Sunitinib malate2.5 µmol/L | -- |
| Genistein | Sotagliflozin 30µmol/L + Genistein 0 | Sotagliflozin 30µmol/L + Genistein 0.1 µmol/L | -- | -- |
| Dacomitinib | Sotagliflozin 30µmol/L + Dacomitinib0 | Sotagliflozin 30µmol/L + Dacomitinib0.1 µmol/L | Sotagliflozin 30µmol/L + Dacomitinib2.5 µmol/L | -- |
| Osimertinib mesylate | Sotagliflozin 30µmol/L + Osimertinib mesylate 0 | Sotagliflozin 30µmol/L + Osimertinib mesylate 0.1 µmol/L | -- | -- |
| Sunitinib | Sotagliflozin 30µmol/L + Sunitinib 0 | Sotagliflozin 30µmol/L + Sunitinib 0.1 µmol/L | Sotagliflozin 30µmol/L + Sunitinib 10 µmol/L | -- |
| Vandetanib | Sotagliflozin 30µmol/L + Vandetanib 0 | Sotagliflozin 30µmol/L + Vandetanib 0.1 µmol/L | Sotagliflozin 30µmol/L + Vandetanib25 µmol/L | Sotagliflozin 30µmol/L + Vandetanib50 µmol/L |
| Brivanib | Sotagliflozin 30µmol/L + Brivanib 0 | Sotagliflozin 30µmol/L + Brivanib 0.1 µmol/L | Sotagliflozin 30µmol/L + Brivanib 10 µmol/L | Sotagliflozin 30µmol/L + Brivanib50 µmol/L |
| Ponatinib | Sotagliflozin 30µmol/L + Ponatinib 0 | Sotagliflozin 30µmol/L + Ponatinib 0.1 µmol/L | -- | -- |
| Varlitinib | Sotagliflozin 30µmol/L + Varlitinib 0 | Sotagliflozin 30µmol/L + Varlitinib 0.1 µmol/L | Sotagliflozin 30µmol/L + Varlitinib10 µmol/L | -- |
| Nintedanib Ethanesulfo nate | Sotagliflozin 30µmol/L + Nintedanib Ethanesulfonate0 | Sotagliflozin 30µmol/L + Nintedanib ethanesulfonate 0.1 µmol/L | Sotagliflozin 30µmol/L + Nintedanib ethanesulfonate 10 µmol/L | -- |
| Nazartinib | Sotagliflozin 30µmol/L + Nazartinib 0 | Sotagliflozin 30µmol/L + Nazartinib 0.1 µmol/L | Sotagliflozin 30µmol/L + Nazartinib 10 µmol/L | -- |
| Cabozantinib | Sotagliflozin 30µmol/L + Cabozantinib 0 | Sotagliflozin 30µmol/L + Cabozantinib 0.1 µmol/L | Sotagliflozin 30µmol/L + Cabozantinib 10 µmol/L | -- |
| ENMD-2076 L-(+)-TART ARIC ACID | Sotagliflozin 30µmol/L + ENMD-2076 L-(+)-TARTARIC ACID0 | Sotagliflozin 30µmol/L + ENMD-2076 L-(+)-TARTARIC ACID 0.1 µmol/L | Sotagliflozin 30µmol/L + ENMD-2076 L-(+)-TARTARIC ACID 10 µmol/L | -- |
| Icotinib | Sotagliflozin 30µmol/L + Icotinib 0 | Sotagliflozin 30µmol/L + Icotinib 2.5 µmol/L | -- | -- |
| Lenvatinib Mesylate | Sotagliflozin 30µmol/L + Lenvatinib Mesylate 0 | Sotagliflozin 30µmol/L + Lenvatinib Mesylate 0.1 µmol/L | Sotagliflozin 30µmol/L + Lenvatinib mesylate 10 µmol/L | Sotagliflozin 30µmol/L + Lenvatinib mesylate 50 µmol/L |
| AZD3759 | Sotagliflozin 30µmol/L + AZD3759 0 | Sotagliflozin 30µmol/L + AZD3759 0.1 µmol/L | Sotagliflozin 30µmol/L + AZD3759 10 µmol/L | Sotagliflozin 30µmol/L + AZD3759 50 µmol/L |
| Lazertinib | Sotagliflozin 30µmol/L + Lazertinib 0 | Sotagliflozin 30µmol/L + Lazertinib 0.1 µmol/L | Sotagliflozin 30µmol/L + Lazertinib 0.5 µmol/L | -- |

The results showed that each combination administration group showed a significantly better inhibitory effect on tumor cells than the single agent control group.

### Example 6

Examples 1 to 4 are all tests at the cell level. In order to further verify the anti-tumor effect in vivo of the diabetes treatment drug Sotagliflozin discovered in the present disclosure and the combination thereof with TKI inhibitor drugs, lung cancer A549 cells and the Balbc nude mice from Charles River Laboratories were used in tumor treatment experiments. After growing to the logarithmic phase, A549 cells were collected and resuspended in serum-free DMEM medium to 5×10⁷ cells per ml. Each mouse was inoculated with 100 µl of 5×10⁶ cells, and the tumor size was measured 19 days later. Mice were grouped according to the tumor size, and the average tumor size in each group was the same. Mice were administered after grouping. According to the current recommended daily dose 200-400 mg of Sotagliflozin for diabetic patients, corresponding to 22 mg to 44 mg per kilogram of body weight for mice, we finally chose a dose of 30 mg/kg of Sotagliflozin for oral administration in mice. The dose of Gefitinib was 100 mg per kilogram according to the reported dose for the treatment of A549 xenograft tumor in previous studies. The two drugs were administered by gavage, consistent with the current oral mode in clinical use. The dosing cycle was once every two days. The tumor size was measured every two days. After 40 days of administration, the test was ended, and the tumor was removed and weighed.

**Table 3 Summary of the efficacy of Sotagliflozin combined with Gefitinib on A549 tumor cell-bearing mice**

| | Average tumor volume | Tumor volume ratio of each group to the control group | Tumor growth inhibition rate | p value |
|---|---|---|---|---|
| Solvent control group | 1034.278808 | | | |
| Sotagliflozin | 430.2976498 | 0.416036417 | 58.39635826 | 0.002 |
| Gefitinib | 504.8082165 | 0.488077502 | 51.19224984 | 0.0012 |
| Sotagliflozin combined with Gefitinib | 211.0447064 | 0.204050112 | 79.59498882 | 0.0001 |

As shown in Figure 7-a~Figure 7-b~Figure 7-c, Sotagliflozin alone and Gefitinib alone can inhibit tumor growth (Figure 7-a~Figure 7-b~Figure 7-c), and the inhibition effect of the combination of Sotagliflozin and Gefitinib on tumors was better than that of the either drug alone. According to the effectiveness evaluation, the tumor growth inhibition rate should be greater than 60%, and the p value should be less than 0.05. The calculated tumor growth inhibition rate and p value of each group are as shown in the above table. Therefore, the administration of Sotagliflozin alone or Gefitinib alone is ineffective, and the administration of the combination of Sotagliflozin and Gefitinib is effective.

The above are the preferred embodiments of the present disclosure.

## Claims

1. Sotagliflozin or a pharmaceutically acceptable salt thereof combined with Gefitinib for the use in treating lung cancer resistant to Gefitinib.

## Patentansprüche

1. Sotagliflozin oder ein pharmazeutisch verträgliches Salz davon in Kombination mit Gefitinib zur Anwendung bei der Behandlung von Gefitinib-resistentem Lungenkrebs.

## Revendications

1. Sotagliflozine ou un de ses sels pharmaceutiquement acceptables, combinée au Géfitinib, destinée à être utilisée dans le traitement du cancer du poumon résistant au Géfitinib.
